# EUROPEAN PATENT SPECIFICATION

(11) **EP 2 483 257 B1**
(45) Date of publication and mention of the grant of the patent: **05.03.2014**
(21) Application number: 10818064.7
(22) Date of filing: 29.09.2010
(51) Int. Cl.: C07D 285/06, C07D 285/36, C07D 309/24, A61K 31/554, A61P 25/00

(54) **2,3,4-BENZOTHIADIAZEPINE-2,2-DIOXIDE DERIVATIVES**
2,3,4-BENZOTHIADIAZEPIN-2,2-DIOXID-DERIVATE
DÉRIVÉS DE 2,3,4-BENZOTHIADIAZÉPINE-2,2-DIOXYDE

(30) Priority: 29.09.2009 HU 0900616
(43) Date of publication of application: 08.08.2012
(73) Proprietor: EGIS GYÓGYSZERGYÁR Nyilvánosan Müködö Részvénytársaság, 1106 Budapest (HU)
(72) Inventor: FETTER, József, H-2100 Gödöllö (HU); BERTHA, Ferenc, H-1171 Budapest (HU); MOLNAR, Balázs, H-2117 Isaszeg (HU); SIMIG, Gyula, H-1126 Budapest (HU); BARKÓCZY, József, H-1016 Budapest (HU); VOLK, Balázs, H -1165 Budapest (HU); LÉVAY, György, H-2092 Budakeszi (HU); GACSÁLYI, István, H-1201 Budapest (HU); GIGLER, Gábor, H-1119 Budapest (HU); KOMPAGNE, Hajnalka, H-1221 Budapest (HU); MARKÓ, Bernadett, H-1171 Budapest (HU); NAGY, Katalin, H-8400 Ajka (HU); KIRICSI, Péter, H-1043 Budapest (HU); HÁRSING, László, Gábor, H-1071 Budapest (HU); SZÉNÁSI, Gábor, H-2096 Üröm (HU)
(74) Representative: Stolmár & Partner
(86) International application number: PCT/HU2010/000103
(87) International publication number: WO 2011/039554

(56) References cited:
- J. F. KING ET AL: "Chlorination of Heterocyclic and Acyclic Sulfonhydrazones", CAN. J. CHEM., vol. 49, no. 6, March 1971 (1971-03), pages 943-955, XP002632360, DOI: 10.1139/v71-154 cited in the application

## Description

### Field of the invention

The invention relates to 2,3,4-benzothiadiazepine-2,2-dioxide derivatives and enantiomers of general Formula **(I),** process for their preparation, medicaments containing said compounds and the use thereof. 2,3,4-Benzothiadiazepine-2,2-dioxide derivatives of general Formula **(I)** are effective in the treatment of disorders of the central nervous system. Further objects of the present invention are the use of the compounds of the general Formula **(I)** in the manufacture of a medicament containing the same as active ingredient.

### Technical background of the invention

The compounds of the general Formula **(I)** of the present invention are new. The most closely related compound known from the prior art is 1,3-dihydro-2,3,4-benzothiadiazepine-2,2-dioxide of Formula **(B)** (J. F. King, A. Hawson, B, L. Huston, L. J. Danks, J. Komery, Can. J. Chem., 1971, 49, 943).

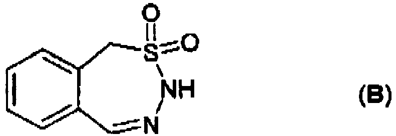

Substituted derivatives of the Formula **(B)** have never been described.

### Summary of the invention

The aim of the present invention is to provide new 2,3,4-benzothiadiazepine-2,2-dioxide derivatives effective in the treatment of disorders of the central nervous system.

The above mentioned aim can be achieved by the new compounds of the general Formula (I), 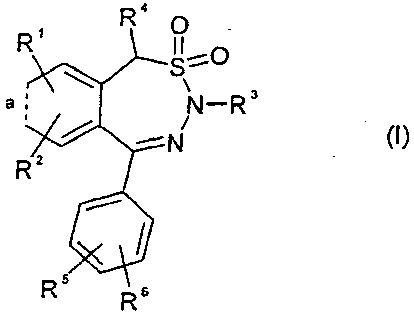 wherein,
- 'a': represents a single or double bond,
- R¹ and R²: represent, independently, a hydrogen atom, halogen atom, cyano group, C₁₋₆ alkyl group, C₁₋₆ alkoxy group trifluoromethyl group or other C₁₋₆ alkyl group substituted by one or more halogen atom
or if
- 'a': represents a single bond,
- R¹ and R²: together form a group of the formula =N-S-N= attached to the atoms of the bond 'a' according to the partial Formula (A)
- R³ and R⁴: represent, independently, a hydrogen atom, C₁₋₆ alkyl group, C₁₋₆ alkenyl group, C₃₋₆ cycloalkyl group, aryl group or aralkyl group,
- R⁵ and R⁶: represent, independently, a hydrogen atom, halogen atom, cyano group, C₁₋₆ alkyl group, C₁₋₆ alkoxy group, trifluoromethyl group or other C₁₋₆ alkyl group substituted by one or more halogen atom
and/or enantiomers and/or pharmaceutically suitable acid addition salts thereof.

A further object of the present invention is to provide a process for the preparation of the compounds of the general Formula (I).

An other object of the invention are pharmaceutical compositions comprising as active ingredient one or more compounds of the general Formula (**I**) as defined in claim 1 and/or enantiomers and/or pharmaceutically acceptable acid addition salts thereof in a therapeutically effective dose together with one or more pharmaceutically applicable diluent, excipient and/or inert carrier.

The present invention further relates to the compounds of the general Formula (I) for the use as a medicament.

The present invention further relates to the use of the compounds of the general Formula (I) in the treatment and/or prevention of the disorder of the central nervous system.

Furthermore our invention relates to compounds of the general Formula (I) for treatment, which comprises administering a patient suffering from a central nervous system disorder, a therapeutically effective dose of one or more compounds of general Formula **(I)** as defined in claim 1 and/or enantiomers and/or pharmaceutically suitable acid additional salts thereof.

### Detailed description of the invention

The present invention relates to compounds of the general Formula **(I)** 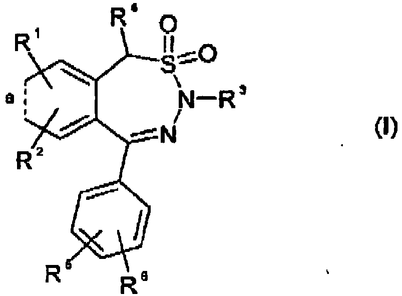 wherein
- 'a': represents a single or double bond,
- R¹ and R²: represents, independently, a hydrogen atom, halogen atom, cyano group, C₁₋₆ alkyl group, C₁₋₆ alkoxy group, trifluoromethyl group, or other C₁₋₆ alkyl group substituted by one or more halogen atom
or if
- 'a': represents a single bond,
- R¹ and R²: together form a group of the formula =N-S-N= attached to the atoms of the bond 'a' according to the partial Formula 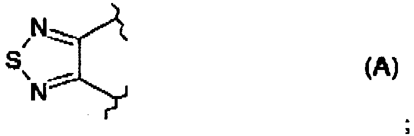
- R³ and R⁴: represent, independently, a hydrogen atom, C₁₋₆ alkyl group, C₁₋₆ alkenyl group, C₃₋₆ cycloalkyl group, aryl group or aralkyl group,
- R⁵ and R⁶: represent, independently, a hydrogen atom, halogen atom, cyano group, C₁₋₆ alkyl group, C₁₋₆ alkoxy group, trifluoromethyl group or other C₁₋₆ alkyl group substituted by one or more halogen atom
and/or enantiomers and/or pharmaceutically suitable acid addition salts thereof.

The definitions of the substituents referred to in the present specification are the following.

The expression "alkyl group" represents a straight or branched saturated hydrocarbon chain having 1-6 carbon atoms, preferably 1-4 carbon atoms, e.g. methyl, ethyl, *n*-propyl, *i*-propyl, *n*-butyl, *i*-butyl, *s*-butyl, *t*-butyl group.

"Alkoxy group" represents a hydroxy group substituted with an above defined alkyl group.

"Alkenyl group" represents a straight or branched-chain hydrocarbon group containing one double bond and having 1-6 carbon atoms, preferably 1-4 carbon atoms.

"Aralkyl group" represents an alkyl group substituted at any position by an aromatic substituent, e.g. an optionally substituted phenyl group. Preferably the aralkyl group is an optionally substituted benzyl group.

"Halogen atom" is a fluorine, chlorine, bromine or iodine atom, preferably chlorine or fluorine.

"Pharmaceutically acceptable" acid addition salts are salts formed with a non-toxic inorganic acid, e.g. hydrochloric acid, sulfuric acid, phosphoric acid, or with a non-toxic organic acid, e.g. acetic acid, fumaric acid, maleic acid, lactic acid, maleic acid, tartaric acid.

Preferable representatives of the compounds of the general Formula (I) are:
8-cyano-5-(4-fluoro-phenyl)-1,3-dihydro-2,3,4-benzothiadiazepine-2,2-dioxide;
7-chloro-5-(3-fluoro-phenyl)-1,3-dihydro-2,3,4-benzothiadiazepine-2,2-dioxide,
9-(4-methyl-phenyl)-5*H*,7*H*-[1,2,5]thiadiazolo[3,4-*h*]-2,3,4-benzothiadiazepine-6,6-dioxide;
9-(4-methoxy-phenyl)-5*H*,7*H-*[1,2,5]thiadiazolo[3,4-*h*][2,3,4]benzothiadiazepine-6,6-dioxide;
9-(3,4-dimethoxy-phenyl)-5*H*,7*H*-[1,2,5]thiadiazolo[3,4-*h*][2,3,4]benzothiadiazepine-6,6-dioxide;
9-(4-fluoro-phenyl)-5*H*,7*H-*[1,2,5]thiadiazolo[3,4-*h*][2,3,4]benzothiadiazepine-6,6-dioxide;
7-chloro-5-(4-fluoro-phenyl)-1,3-dihydro-2,3,4-benzothiadiazepien-2,2-dioxide;
7-fluoro-5-(4-fluoro-phenyl)-1,3-dihydro-2,3,4-benzothiadiazepine-2,2-dioxide;
7,8-dichloro-5-(4-fluoro-phenyl)-1,3-dihydro-2,3,4-benzothiadiazepine-2,2-dioxide;
7-chloro-5-(4-fluoro-phenyl)-3-methyl-1,3-dihydro-2,3,4-benzothiadiazepine-2,2-dioxide;
7-chloro-3-ethyl-5-(4-fluoro-phenyl)-1,3-dihydro-2,3,4-benzothiadiazepine-2,2-dioxide;
7-chloro-5-(4-fluoro-phenyl)-3-propyl-1,3-dihydro-2,3,4-benzothiadiazepine-2,2-dioxide;
7-chloro-5-(4-fluoro-phenyl)-3-(prop-2-inyl)-1,3-dihydro-2,3,4-benzothiadiazepine-2,2-dioxide;
7-chloro-5-(4-fluoro-phenyl)-3-isopropyl-1,3-dihydro-2,3,4-benzothiadiazepine-2,2-dioxide;
7-chloro-5-(4-fluoro-phenyl)-3-(3-methyl-but-2-enyl)-1,3-dihydro-2,3,4-benzothiadiazepine-2,2-dioxide;
7-chloro-5-(4-fluoro-phenyl)-3-(3-methyl-butyl)-1,3-dihydro-2,3,4-benzothiadiazepine-2,2-dioxide;
3-benzyl-7-chloro-5-(4-fluoro-phenyl)-1,3-dihydro-2,3,4-benzothiadiazepine-2,2-dioxide;
7-fluoro-5-(4-fluoro-phenyl)-3-methyl-1,3-dihydro-2,3,4-benzothiadiazepine-2,2-dioxide;
7-fluoro-3-ethyl-5-(4-fluoro-pbenyl)-1,3-dibydro-2,3,4-benzothiadiazepine-2,2-dioxide;
3-butyl-7-fluoro-5-(4-fluoro-phenyl)-1,3-di.hydro-2,3,4-benzothiadiazepine-2,2-dioxide;
7-fluoro-5-(4-fluoro-phenyl)-3-(3-methyl-but-2-enyl)-1,3-dihydro-2,3,4-benzothiadiazepine-2,2-dioxide;
7-fluoro-5-(4-fluoro-phenyl)-3-(3-methyl-butyl)-1,3-dihydro-2,3,4-benzothiadiazepine-2,2-dioxide;
7-fluoro-5-(4-fluoro-phenyl)-3-hexyl-1,3-dihydro-2,3,4-benzothiadiazepine-2,2-dioxide;
3-benzyl-7-fluoro-5-(4-fluoro-phenyl)-1,3-dihydro-2,3,4-benzothiadiazepine-2,2-dioxide;
7-fluoro-5-(4-fluoro-phenyl)-3-(3-phenyl-propyl)-1,3-dihydro-2,3,4-benzothiadiazepine-2,2-dioxide;
5-(3-fluoro-phenyl)-7-chloro-3-methyl-1,3-dihydro-2,3,4-benzothiadiazepine-2,2-dioxide;
8-cyano-5-(4-fluoro-phenyl)-3-methyl-1,3-dihydro-2,3,4-benzothiadiazepine-2,2-dioxide;
8-fluoro-5-(4-fluoro-phenyl)-3-methyl-1,3-dihydro-2,3,4-benzothiadiazepine-2,2-dioxide;
5-(4-fluoro-phenyl)-8-chloro-3-methyl-1,3-dihydro-2,3,4-benzothiadiazepine-2,2-dioxide;
5-(4-fluoro-phenyl)-3-methyl-8-trifluormethyl-1,3-dihydro-2,3,4-benzothiadiazepine-2,2-dioxide;
7,8-dichloro-5-(4-fluoro-phenyl)-3-methyl-1,3-dihydro-2,3,4-benzothiadiazepine-2,2-dioxide;
9-(4-fluoro-phenyl)-7-methyl-5*H*,7*H*-[1,2,5]thiadiazolo[3,4-*h*][2,3,4]benzothiadiazepine-6,6-dioxide;
7,8-dichloro-3-ethyl-5-(4-fluoro-phenyl)-1,3-dihydro-2,3,4-benzothiadiazepine-2,2-dioxide;
7-chloro-5-(4-fluoro-phenyl)-1,3-dimethyl-1,3-dihydro-2,3,4-benzothiadiazepine-2,2-dioxide;
7-chloro-1-ethyl-5-(4-fluoro-phenyl)-3-methyl-1,3-dihydro-2,3,4-benzothiadiazepine-2,2-dioxide
and/or enantiomers and/or pharmaceutically suitable acid addition salts thereof.

The invention further relates to enantiomers of the compounds of the general Formula **(I)** and the mixtures thereof.

A further object of the present invention is to provide a process for the preparation of the compounds of the general Formula **(I),** which consist of simple reaction steps providing high yield.

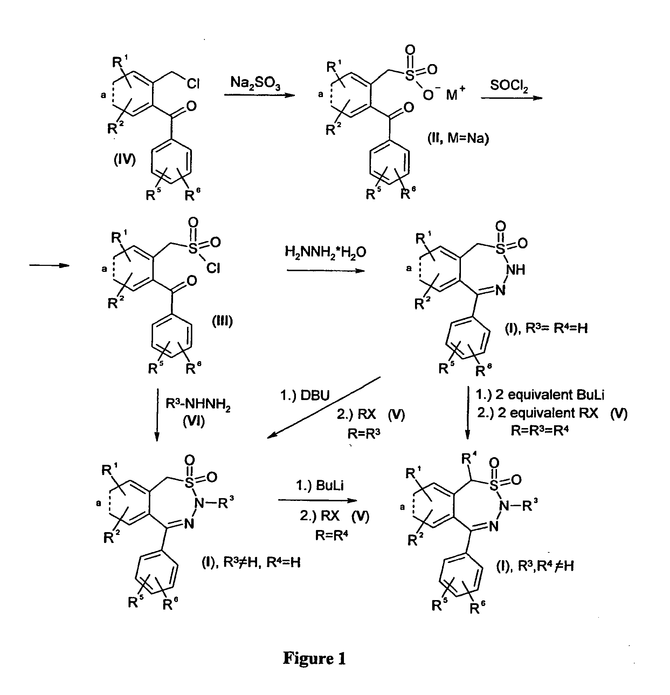

The reaction method used in the preparation of the compounds of the present invention is demonstrated in **Reaction Scheme 1 (****Figure 1****).**

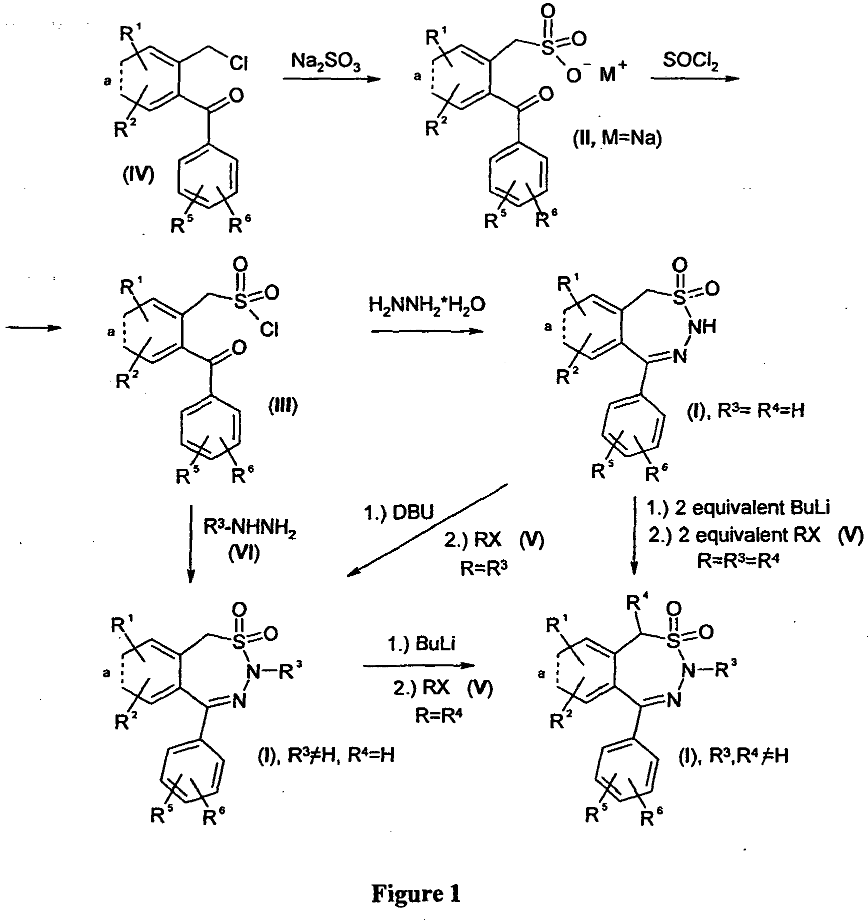

The compounds of the general Formula **(I)** of the present invention, wherein R³ and R⁴ are hydrogen atoms and R¹, R², R⁵ and R⁶ are defined in claim 1 **(I,** R³=R⁴=H) and pharmaceutically suitable acid addition salts thereof are prepared by reacting the compound of the Formula **(II),** wherein R¹, R², R⁵ and R⁶ are defined in claim 1 and M represents monovalent or multivalent metals, preferably alkali metals, more preferably a sodium atom, with tionyl chloride, and subsequently the obtained compound of the general Formula **(III),** wherein R¹, R², R⁵ and R⁶ are defined in claim 1, is reacted with hydrazine.

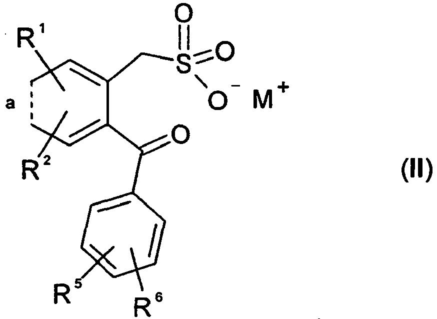

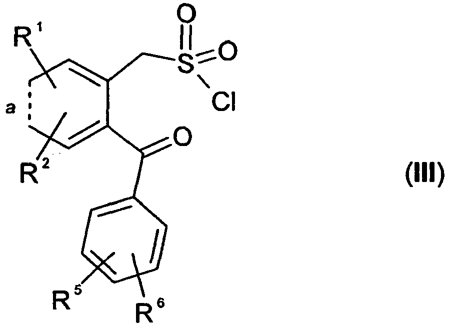

The compounds of the general Formula **(I)** of the present invention, wherein R⁴ is hydrogen and R¹, R², R⁵, R⁶ and R³ are defined in claim 1 provided that R³ is different from hydrogen **(I,** R³≠H, R⁴=H) and pharmaceutically suitable acid addition salts thereof are prepared by reacting the 2,3,4-benzothiadiazepinedioxide derivatives of the general Formula **(I),** wherein R³ and R⁴ are hydrogen, R¹, R², R⁵ and R⁶ are defined in claim 1 **(I,** R³=R⁴=H), with agents suitable for introducing an alkyl group, alkenyl group or aralkyl group, in the presence of a base.

Furthermore, the compounds of the general Formula **(I)** of the present invention, wherein R⁴ is hydrogen and R¹, R², R⁵, R⁶ and R³ are defined in claim 1 provided that R³ is different from hydrogen **(I,** R³≠H, R⁴=H) and pharmaceutically suitable acid addition salts thereof can be prepared by reacting compounds of the general formula **(III),** wherein R¹, R², R⁵ and R⁶ are defined in claim 1, with a substituted hydrazine of general formula **(VI),** wherein R³ is defined in claim 1.

R³-NH-NH₂ **(VI)**

The compounds of the general Formula **(I)** of the present invention, wherein R³ and R⁴ are defined in claim 1 provided that R³ and R⁴ are identical and different from hydrogen and R¹, R², R⁵ and R⁶ are defined in claim 1 **(I,** R³=R⁴≠H) and pharmaceutically suitable acid addition salts thereof are prepared by reacting the 2,3,4-benzothiadiazepine-dioxide derivatives of the general Formula **(I),** wherein R³ and R⁴ are hydrogen, R¹, R², R⁵ and R⁶ are defined in claim 1 **(I,** R³=R⁴=H), with agents suitable for introducing an alkyl group, alkenyl group or aralkyl group, in the presence of a base.

Furthermore, the compounds of the general Formula **(I)** of the present invention, wherein R³ and R⁴ are defined in claim 1 provided that R³ and R⁴ are different from hydrogen and R¹, R², R⁵ and R⁶ are defined in claim 1 **(I,** R³≠H, R⁴≠H) and pharmaceutically suitable acid addition salts thereof can be prepared by reacting the 2,3,4-benzothiadiazepine-dioxide derivatives of the general Formula **(I),** wherein R⁴ is hydrogen, R¹, R², R⁵, R⁶ and R³ are defined in claim 1 provided that R³ is different from hydrogen **(I,** R³≠H, R⁴=H), with agents suitable for introducing an alkyl group, alkenyl group or aralkyl group, in the presence of a base.

Further objects of the present invention are the compounds of the general Formula **(III),** 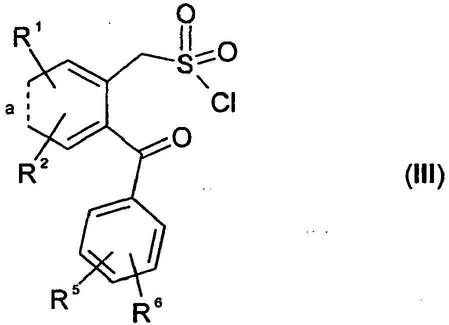 and the compounds of the general Formula **(II),** 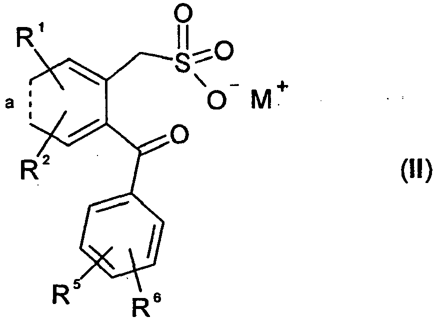 wherein M represents monovalent or multivalent metals, preferably alkali metals more preferably a sodium atom.

Preferred representatives of the compounds of the general Formula **(II)** are:
sodium [4-chloro-2-(4-fluoro-benzoyl)-phenyl]-methanesulfonate;
sodium [6-[(3,4-dimethoxy-phenyl)-carbonyl]-2,1,3-benzothiadiazole-5-yl]-methanesulfonate;
sodium [6-[(4-fluoro-phenyl)-carbonyl]-2,1,3-benzothiadiazole-5-yl]-methanesulfonate;
sodium [6-[(4-methoxy-phenyl)-carbonyl]-2,1,3-benzothiadiazole-5-yl]-methanesulfonate.

Further object of the present invention is the for the preparation of the compounds of general Formula **(III),** which comprises reacting the compound of general Formula **(II),** wherein R¹, R², R⁵ and R⁶ are defined in claim 1 with thionyl chloride.

Another object of the present invention the process for the preparation of the derivatives of general Formula **(II),** wherein R¹, R², R⁵ and R⁶ are defined in claim 1, which comprises reacting the compound of general Formula **(IV),** wherein R¹, R², R⁵ and R⁶ are defined in claim 1 with ammonium sulfite or alkali sulfite in the present of water or water-immiscible solvent, preferably in acetone, dioxane or in an aliphatic alcohol comprising 1 to 4 carbon atoms or in the mixture thereof.

### Compounds of the Formula (IV)

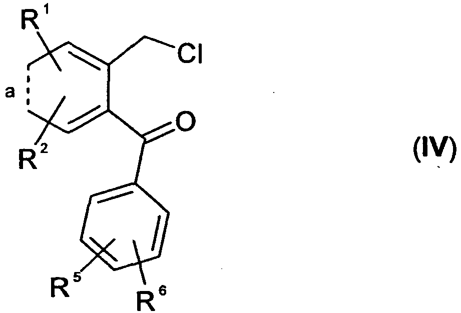

were prepared by a method known from the art for the synthesis of compounds with a similar structure (J. Org. Chem, 1988, 53, 3621; J. Org. Chem., 2003, 68, 4123). The [substituted 2-(substituted arylcarbonyl)-phenyl]methanesulfonate salts of the Formula **(II)**
are prepared by the replacement of the halogen atom to sulfonic acid moiety in water, a water-miscible solvent (acetone, dioxane, alcohols) or in a mixture of water and a water-miscible solvent, by reacting the halogen compound with ammonium or alkali sulfites.

The compounds of Formula **(II)** are new and have not been described in the literature before. The metal salts of the general Formula **(II)** are formed by monovalent or multivalent metals, preferably alkali metals more preferably sodium atom.

The sulfonyl chlorides of the Formula **(III)** are prepared from the new benzylsulfonyl derivatives of the Formula **(II)** with tionyl chloride. The 2,3,4-benzothiadiazepine-2,2-dioxide derivatives of the general Formula **(I),** wherein R³ and R⁴ represent hydrogen atom, are obtained from sulfonyl chlorides of the Formula **(III)** by closing the ring with hydrazine. Preferably the compounds of Formula **(III)** are not purified, but the crude product was reacted with hydrazine immediately.

An alternative one-pot method involves ring closure of sulfonyl chlorides of Formula **(III)** by reacting with compound of Formula **(VI),**

**R³-NH-NH₂** **(VI)**

wherein R³ represents alkyl, alkenyl or aralkyl group. This one-pot method yields 2,3,4-benzothiadiazepine-2,2-dioxide derivatives of the Formula **(I),** wherein R³ is an alkyl, alkenyl or aralkyl group and R⁴ represents hydrogen.

Optionally the 2,3,4-benzothiadiazepine-2,2-dioxide derivatives of the Formula **(I),** wherein R³ and R⁴ represent hydrogen, can be converted to compounds of the Formula **(I),** wherein R³ represents alkyl group, alkenyl group or aralkyl group and R⁴ represents hydrogen atom, by reacting with an agent suitable for introducing an alkyl, alkenyl group or aralkyl group, in the presence of a base. In the reaction the agent suitable for introducing an alkyl group, alkenyl group or aralkyl group is the alkylating agent of the Formula **(V)**

**RX** **(V)**

wherein R represents R³ substituent of the end-product as defined in claim 1, X represents a leaving group, e.g. halogen atom, methanesulfonyloxy group or *p-*toluenesulfonyloxy group, preferably halogen atom, more preferably chlorine, bromine, or iodine atom. The base used is an organic or inorganic base, e.g. alkali metal carbonate, alkali metal hydrogencarbonate, aliphatic or alicyclic secondary or tertiary amines, preferably 1,8-diazabicyclo[5.4.0]undec-7-ene (DBU).

If desired, the 2,3,4-benzothiadiazepine-2,2-dioxide derivatives of Formula **(I),** wherein R³ represents alkyl group, alkenyl group or aralkyl group and R⁴ represents hydrogen, can be converted to compounds of Formula **(I),** wherein R³ and R⁴ represents alkyl group, alkenyl group or aralkyl group, by reacting with an alkylating agent of the Formula (V), wherein R represents R⁴ substituent of the end-product as defined in claim 1, in the presence of a strong base. The strong base is an organic or inorganic base, e.g. alkali metal alkoxide, alkali metal hydride, alkaline earth metal hydride or alkyllithium compound, preferably alkyl lithium compound, more preferably butyllithium.

There is a one-pot method, wherein the 2,3,4-benzothiadiazepine-2,2-dioxide derivatives of Formula **(I),** wherein R³ and R⁴ represents hydrogen can be converted directly to compounds of Formula **(I)** wherein R³ and R⁴ represent the same alkyl group, alkenyl group or aralkyl group by reacting with alkylating agent of the Formula **(V)** in the presence of a strong base.

In this case the alkylating agent of the Formula **(V)** as defined above is used in 2 molar equivalent amount relative to the molar amount of the starting compound. The strong base in the alkylation reaction is organic or inorganic base in 2 molar equivalent amount, e.g. alkali metal alkoxide, alkali metal hydride, alkaline earth metal hydride or alkyl lithium compound, preferably alkyllithium compound, more preferably butyllithium.

Further objects of the present invention are pharmaceutical compositions comprising as active ingredient one or more compounds of the general Formula **(I)** as defined in claim 1 and/or enantiomers and/or pharmaceutically acceptable acid-addition salts thereof in a therapeutically effective dose together with one or more pharmaceutically applicable diluent, excipient and/or inert carrier.

Pharmaceutical compositions of the present invention contain in general 0.1 to 95.0 per cent by weight, preferably 1.0 to 50.0 per cent by mass, more preferably 5.0 to 30.0 per cent by mass of active ingredient.

The pharmaceutical compositions of the present invention are suitable for oral (e.g. powders, tablets, film-coated tablets, capsules, microcapsules, drops/pills, solutions, suspensions or emulsions), parenteral (e.g. in form of intravenous, intramuscular, subcutaneous or intraperitoneal injections or infusion compositions), rectal (e.g. in form of suppositories), transdermal (e.g. patches) administration, or can be used in the form of implants or for local treatment (e.g. ointment, gels or patches).

The solid, semisolid or liquid pharmaceutical dosage forms of the present invention are known per se, the methods of manufacturing the compositions are known in the state of the art.

The solid pharmaceutical compositions suitable for oral administration may be powders, capsules, tablets, film-coated tablets, microcapsules etc., and optionally comprise binding agents such as gelatine, sorbitol, poly(vinyl-pyrrolidone) etc.; filling agents such as lactose, glucose, starch, calcium phosphate etc.; auxiliary substances for tabletting such as magnesium stearate, talc, polyethylene glycol, silica etc.; wetting agents such as sodium laurylsulfate etc.

The liquid pharmaceutical compositions suitable for oral administration may be solutions, suspensions or emulsions and can comprise e.g. suspending agents such as gelatine, carboxymethylcellulose etc.; emulsifiers such as sorbitane monooleate etc.; solvents such as water, oils, glycerol, propyleneglycol, ethanol etc.; preservatives such as methy p-hydroxybenzoate etc. and a the carrier.

Typical parenteral compositions consisting of a solution or suspension of the compound of Formula **(I)** and/or enantiomers and/or pharmaceutically suitable acid additional salts thereof in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

Compositions of the present invention for nasal administration containing a compound of Formula **(I)** and/or enantiomers and/or pharmaceutically suitable acid additional salts thereof may conveniently be formulated as aerosols, drops, gels and powders.

Aerosol formulations of the present invention typically comprise a solution or fine suspension of the compound of Formula **(I)** and/or enantiomers and/or pharmaceutically suitable acid additional salts thereof in a physiologically acceptable aqueous or non-aqueous solvent and are usually presented in a single or multidose quantities in sterile form in a sealed container, which can take the form of a cartridge or refill for use with an atomizing device. Alternatively, the sealed container may be a unitary dispensing device, such as a single dose nasal inhaler or an aerosol dispenser fitted with a metering valve which is intended for disposal once the contents of the container have been exhausted. Where the dosage form comprises an aerosol dispenser, it will contain a propellant which can be a compressed gas, such as compressed air or an organic propellant, such as a fluorochlorohydrocarbon. The administration of aerosol dosages can also take the form of a pump-atomiser.

Compositions of the present invention containing a compound of Formula **(I)** and/or enantiomers and/or pharmaceutically suitable acid additional salts thereof are suitable for buccal or sublingual administration including tablets, lozenges and pastilles, wherein the active ingredient is formulated with a carrier, such as sugar and acacia, tragacanth, or gelatine, glycerol, etc.

Compositions of the present invention containing a compound of Formula **(I)** and/or enantiomers and/or pharmaceutically suitable acid additional salts thereof for rectal administration are conveniently in the form of suppositories containing a conventional suppository base, such as cocoa butter, polyethylene glycol or other materials commonly used in the art. The suppositories may be conveniently formed by first admixing the composition with the softened or melted carrier(s) followed by chilling and shaping in moulds.

Compositions of the present invention containing a compound of Formula (**I**) and/or pharmaceutically suitable acid additional salts thereof for transdermal administration include ointments, gels and patches.

Dosage forms listed above as well as other dosage forms are known per se, see e. g. Remington's Pharmaceutical Sciences, 18th Edition, Mack Publishing Co., Easton, USA (1990).

The compositions containing the compounds of the general Formula **(I)** of the present inventions contain the active ingredient in form of dose units.

The typical dosage for adult patients is 0,1-1000 mg of a compound of Formula **(I)** or an enantiomer or a pharmaceutically acceptable acid additional salt thereof, which can be administered once or in portions. The actual dose depends on many factors and its determination of is a medical task.

A further subject of the invention are compounds defined in claims 1 for use as a medicament and compounds of general Formula (I) and/or enantiomers and/or pharmaceutically suitable acid additional salts thereof for use as a medicament in the treatment of diseases of the central nervous system. Furthermore our invention relates to compounds of the general Formula (I) for treatment, which comprises administering a patient suffering from a central nervous system disorder a therapeutically effective dose of one more compounds of general Formula **(I)** as defined in claim **1** and/or enantiomers and/or pharmaceutically suitable acid additional salts thereof.

In the investigations of biological efficiency of the compounds of general Formula **(I)** as defined in claim 1, it has been found that the compounds are effectively modulating central nervous system functions, thus, it is expected that the compounds of the invention are well suited for the prevention and/or treatment of several diseases of the central nervous system.

The *in vivo* biological effectiveness of the compounds of the present invention on the central nervous system was examined by the following experimental model.

### Changes in amino acid neurotransmitter levels in the cortical area of male rats

Male SPDR rats weighing 200-250 g, were used in the experiments. Changes in the concentration of amino acid neurotransmitters were measured from cortical areas by high-performance liquid chromatography (HPLC) and electrochemical detection.

The investigations were carried out with two experimental groups wherein each group contained 8 animals. One group was administered intraperitoneally by vehicle only (control group). The other group was administered intraperitoneally by the compound of the Example 15, suspended in vehicle. The dose of administration was in the form of 0.4 % aqueous methylcellulose suspension.

The animals were decapitated 30 minutes after the administration, brains have been removed and required brain regions were dissected. The tissues were stored at -70 °C.

The tissues were homogenised in ice-cold 0.1 M perchlorate in 500 volume excess. The homogenates were intensively mixed, centrifuged at 4 °C at 20,000 rpm for 20 minutes, the supernatants were removed and an aliquot thereof was immediately injected after derivatisation without further sample purification.

The automatic derivatisation was carried out at 25 °C using *o-*phtaldialdehyde/Na₂SO₃ reagent; the reaction time was 10 minutes.

The assay was performed using an Agilent 1100 HPLC, separation was carried out by gradient elution on an Agilent Zorbax SB-C18 (250 × 4,6 mm, 5 µm) column, utilising 0,1 M NaH₂PO₄, pH=4,5/ methanol eluent.

For electrochemical detection, ESA Coulochem III detector was used (analytical cell: ESA 5011A), the potential of working electrode was +650 mV.

The tissue concentrations of the amino acids were measured by standard solutions containing amino acids with known concentration.

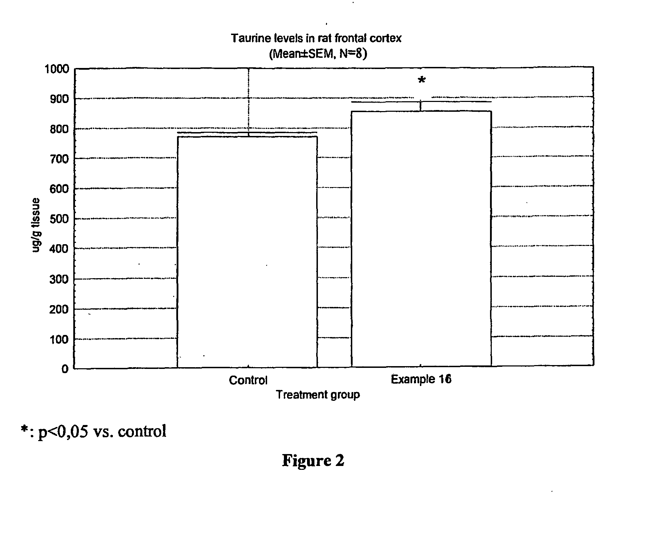

It has been found that concentrations of the amino acid taurine were significantly increased in the frontal cortex of the animals administered with the compound of Example 16 of the present invention, compared to frontal cortex obtained from animals of the control group. Figure 2 demonstrates the results.

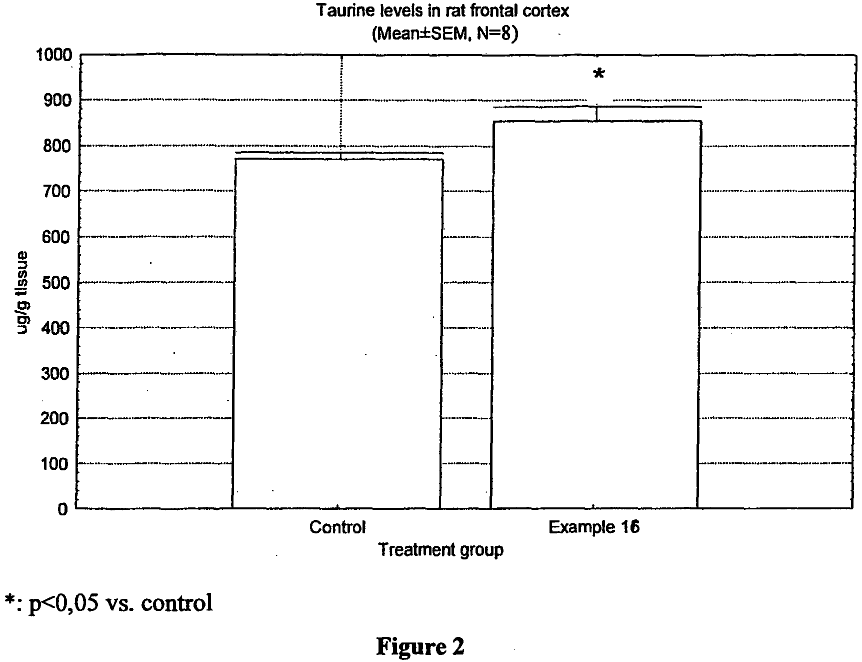

It is known from the literature that taurine, which is non-proteinaceous amino acid, occurs in high quantities in the central nervous system *(*Oja and Saransaari, Proc. West. Pharmacol. Soc., 50: 8-15, 2007*.).* Taurine plays important role in several neurophysiologic processes. It is also known that taurine possesses antioxidant properties and regulates the intracellular calcium level, thus modulates neuronal receptor function *(*Szymansky and Winiarska, Postepy. Hig. Med Dosw., 62: 75-86, 2008*.).* The extent of long-term potentiation (LTP) in striatum decreased in taurine transporter knock-out mice and there were pathological changes in neuroreceptor expression *(*Warskulat és mtsai, Methods Enzymol., 428: 439-58, 2007*.).* These results support the important modulator role of taurine in the central nervous system.

It has been surprisingly found that compounds of the present invention induce a statistically significant increase in the amount of taurine in the examined brain regions, resulting in the effective modulation of neuronal functions by the taurine pathway.

Therefore, it is expected that the compounds of the present invention are well suited for the prevention and/or treatment of several diseases of the central nervous system. Due to this surprising effect, it has been recognized that the compounds of the present invention are able to modulate the active function of central nervous system, normalize the pathological features, thus are expected to be effective in the treatment of disorders of the central nervous system.

The invention is further elucidated by means of following Examples.

### Examples:

### Halogen - sulfite exchange (Process "A")

To the solution of 540 ml dioxane and 800 ml water, 0.27 mol of (substituted 2-chloromethyl-phenyl)-(substituted aryl)-methanone and 0.36 mol of sodium sulfite are added and boiled for ca. two hours. After cooling ca. 800 ml solvent is evaporated from the reaction mixture.

Process A/1: the residual aqueous phase is extracted with ethyl acetate or *tert*-butyl methyl ether and the organic phase is extracted with 2x80 ml 1M NaOH solution. The combined alkaline and aqueous phase is decoloured with activated carbon and evaporated to ca. 250 ml. Thus an oily product is obtained. 52 ml of concentrated hydrochloric acid is added to the mixture under cooling and stirring and the reaction mixture is stirred for half an hour. The precipitated substance is filtered, washed and dried (sodium salt).

Process A/2: If the evaporation of the aqueous phase results in sulfonic acid crystals, the crystals are filtered, washed and dried.

### Example 1

### Sodium [4-chloro-2-(4-fluoro-benzoyl)-phenyl]-methanesulfonate

The title product is prepared according to the process A/1 starting from (5-chloro-2-chloromethyl-phenyl)-(4-fluorophenyl)-methanone.
Yield: 60 %
Melting point: 254-256 °C (water)
IR (KBr): 3448, 1667, 1598, 1412 cm^{-1.}
HNMR (DMSO-*d*₆, 500 MHz): 7.80 (dd, J=8.8, 5.7 Hz, 2H), 7.55 (m, 2H), 7.35 (t, J=8.8 Hz, 2H), 7.23 (m, 1H), 3.98 (s, 2H) ppm.
CNMR (DMSO-*d*₆,125 MHz): 194.9, 165.1 (d, J = 251.5 Hz), 141.0, 134.6, 134.0 (d, J=2.9 Hz), 133.6, 133.4 (d, J=9.3 Hz), 130.8, 129.9, 127.9, 115.5 (d, J=22.0 Hz), 53.0 ppm.

Elemental analysis [calculated on the basis of the Formula C₁₄H₉ClFO₄SNa (350.73)]

| | |
|---|---|
| Calculated: | C 47.94, H 2.59, Cl 10.11, S 9.14 % |
| Measured: | C 47.56, H 2.77, Cl 10.00, S 9.06 % |

Gravimetric Na content: 6.22% (calculated: 6.55%)

### Example 2

### Sodium [4,5-dichloro-2-(4-fluoro-benzoyl)-phenyl]-methanesulfonate

The title product is prepared according to the process A/1 starting from (4,5-dichloro-2-chloromethyl-phenyl)-(4-fluorophenyl)-methanone.
Yield: 70 %
Melting point: 216-230 °C (water)
IR (KBr): 3456, 1758, 1667, 1598, 1506, 1285, 1225, 1149, 1044 cm⁻¹.
HNMR (DMSO-*d*₆, 500 MHz): 7.79 (dd, J=8.8, 5.6 Hz, 2H), 7.75 (s, 1H), 7.46 (s, 1H), 7.35 (t, J=8.9 Hz, 2H), 3.96 (s, 2H) ppm.

### Example 3

### Sodium [6-[(3,4-dimethoxyphenyl)-carbonyl]-2,1,3-benzothiadiazol-5-yl]-methanesulfonate

The title product is prepared according to the process A/2 starting from [6-(chloromethyl)-2,1,3-benzothiadiazol-5-yl] (3,4-dimethoxyphenyl)-methanone.
Yield: 86 %
Melting point: 282-283 °C (water)
HNMR (DMSO-*d*₆, 500 MHz): 8.15 (d, J = 0.6 Hz, 1H), 7.91 (d, J = 0.5 Hz, 1H), 7.49 (d, J = 2.0 Hz, 1H), 7.36 (dd, J = 2.0, 8.4 Hz, 1H), 7.07 (d, J=8.5 Hz, 1H), 4.13 (s, 2H), 3.87 (s, 3H), 3.81 (s, 3 H) ppm.

### Example 4

### Sodium [6-[(4-fluorophenyl)-carbonyl]-2,1,3-benzothiadiazol-5-yl]-methanesulfonate

The title product is prepared according to the process A/2 starting from [6-(chloromethyl)-2,1,3-benzothiadiazol-5-yl](4-methoxyphenyl)-methanone.
Yield: 90 %
Melting point: 320-323 °C (water), with decomposition
IR (KBr): 3527, 1657, 1600, 1207, 1044 cm⁻¹.
HNMR (DMSO-*d*₆, 500 MHz): 8.09 (d, J = 0.6 Hz, 1H), 7.91 (d, J = 0.6 Hz, 1H), 7.90 (dd, J = 8.8, 5.5 Hz, 2H), 7.36 (t, J = 8.9 Hz, 2H), 4.21 (s, 2H) ppm.
CNMR (DMSO-*d*₆, 125 MHz): 194.89, 165.10 (d, J = 251.5 Hz), 154.62, 152.35, 141.24, 136.54, 134.03 (d, J = 2.9 Hz), 133.76 (d, J = 9.3 Hz), 124.14, 121.50, 115.32 (d, J = 22.0 Hz), 53.74 ppm.

Elemental analysis [calculated on the basis of the Formula C₁₄H₈FN₂O₄S₂Na (374.35)]

| | |
|---|---|
| Calculated: | C 44.92, H 2.15, N 7.48 % |
| Measured: | C 44.59, H 2.16, N 7.23 % |

### Example 5

### Sodium [6-[(4-methozyphenyl)-carbonyl]-2,1,3-benzothiadiazol-5-yl]-methanesulfonate

The title product is prepared according to the process A/2 starting from [6-(chloromehyl)-2,1,3-benzothiadiazol-5-yl](4-methoxyphenyl)methanone
Yield: 79 %
Melting point: 287-288 °C (water), with decomposition
IR (KBr): 3527, 1647, 1598, 1300cm⁻¹.
HNMR (DMSO-*d*₆, 125 MHz): 8.13 (s, 1H), 7.89 (s, 1H), 7.80 (d, J=9.2 Hz, 2H), 7.06 (d, J=9.2 Hz, 2H), 4.16 (s, 2H), 3.87 (s, 3H) ppm.

### Ring closing reaction (process "B")

78 mmol of sodium [2-(substituated-benzoyl)-substituted phenyl]-methanesulfonate and 100 ml thionyl chloride are boiled for 4 hours. The reaction mixture is evaporated. The residue is dissolved in 250 ml of dichloromethane and the solution is added dropwise to the solution of 750 ml dichloromethane and 75 ml hydrazine under cooling and vigorous stirring. The mixture is stirred for 1.5 hours without cooling. The solution is acidified under ice-cooling with 160 ml cc. hydrochloric acid (to pH=1). The phases are separated and the aqueous phase is extracted with 2×100 ml dichloromethane. The combined aqueous layer is dried over MgSO₄ and evaporated.

Process B/1: the crystalline residue is triturated with mixture of 40 ml ethanol and 30 ml hexane, filtered, washed with 2x20 ml cold ethanol, and dried.

Process B/2: the residue is subjected to flash chromatographic purification with the appropriate solvent and the product is obtained by evaporation.

### Example 6

### 8-Cyano-5-(4-fluoro-phenyl)-1,3-dihydro-2,3,4-benzothiadiazepin-2,2-dioxide

The title product is prepared according to the process B/1 starting from sodium [5-cyano-2-(4-fluoro-benzoyl)-phenyl]-methanesulfonate.
Yield: 35 %
Melting point: 218-219 °C (ethanol)
IR (KBr): 3215, 1601, 1510, 1325, 1150 cm⁻¹.
HNMR (DMSO-*d*₆, 200 MHz): 10.52 (bs, 1H), 8.16 (d, J=1.5 Hz, 1H), 7.96 (dd, J=8.2, 1.8 Hz, 1H), 7.67 (dd, J=9.2, 5.8 Hz, 2H), 7.44 (d, J=7.9 Hz, 1H), 7.34 (t, J=8.9 Hz, 2H), 4.83 (s, 2H) ppm.

Elemental analysis [calculated on the basis of the Formula [C₁₅H₁₀FN₃O₂S (315.33)]

| | |
|---|---|
| Calculated: | C 57.14, H 3.20, N 13.30 % |
| Measured: | C 57.89, H 3.39, N 13.29 % |

### Example 7

### 7-Chloro-5-(3-fluoro-phenyl)-1,3-dihydro-2,3,4-benzothiadiazepin-2,2-dioxide

The title product is prepared according to the process B/1 starting from sodium [4-chloro-2-(3-fluoro-benzoyl)-phenyl]-methanesulfonate.
Yield: 42 %
Melting point: 195-198 °C (ethanol)
IR (KBr): 3200, 1320, 1159 cm⁻¹.
HNMR (CDCl₃, 200 MHz): 7.58 (dd, J=8.1, 2.2 Hz, 1H), 7.46 (d, J=8.4 Hz, 1H), 7.19-7.50 (m, 5H), 7.14 (bs, 1H), 4.40 (s, 2H) ppm.

Elemental analysis [calculated on the basis of the Formula C₁₄H₁₀ClFN₂O₂S (324.76)]

| | |
|---|---|
| Calculated: | C 51.78, H 3.10, Cl 10.92, N 8.63 % |
| Measured: | C 52.13, H 3.09, Cl 10.85, N 8.44 % |

### Example 8

### 9-(4-Methylphenyl)-5H,7H-[1,2,5]thiadiazolo[3,4-h]-2,3,4-benzothiadiazepin-6,6-dioxide

The title product is prepared according to the process B/1 starting from sodium [6-[(4-methoxyphenyl)-carbonyl]-2,1,3-benzothiadiazol-5-yl]-methanesulfonate.
Yield: 70 %
Melting point: 212-215 °C (ethyl acetate)
IR (KBr): 3095, 1325, 1150 cm⁻¹.
HNMR (CDCl₃, 125 MHz): 10.26 (s, 1H), 8.38 (d, J=0.6 Hz, 1H), 7.95 (s, 1H), 7.62 (d, J=8.2, 2H), 7.32 (d, J=8.1 Hz, 2H), 4.92 (s, 2H), 2.39 (s, 3H) ppm.

Elemental analysis [calculated on the basis of the Formula C₁₅H₁₂N₄O₂S₂ (344.42)]

| | |
|---|---|
| Calculated: | C 52.31, H 3.51, N 16.27 % |
| Measured: | C 51.98, H 3.45, N 16.32 % |

### Example 9

### 9-(4-Methoxyphenyl)-5H,7H-[1,2,5]thiadiazolo[3,4-h] [2,3,4]benzothiadiazepin-6,6-dioxide

The title product is prepared according to the process B/1 starting from sodium - [6-(4-methoxy-benzoyl)-benzo[1,2,5]thiadiazol-5-il]-methanesulfonate.
Yield: 68 %
Melting point: 225-227 °C (ethyl acetate)
IR (KBr): 3101, 1601, 1321, 1148 cm⁻¹.
HNMR (CDCl₃, 200 MHz): 10.19 (bs, 1H), 8.37 (s, 1H), 7.98 (s, 1H), 7.70 (d, J= 8.8 Hz, 2H), 7.05 (d, J=8.8 Hz, 2H), 4.92 (s, 2H), 3.84 (s, 3H) ppm.

Elemental analysis [calculated on the basis of the Formula C₁₅H₁₂N₄O₃S₂ (360.42)]

| | |
|---|---|
| Calculated: | C 49.99, H 3.36, N 15.55 % |
| Measured: | C 49.11, H 3.45, N 15.29 % |

### Example 10

### 9-(3,4-Dimethoxyphenyl)-5H,7H-[1,2,5]thiadiazolo[3,4-h] [2,3,4] benzothiadiazepin-6,6-dioxide

The title product is prepared according to the process B/1 starting from sodium [6-(3,4-dimethoxy-benzoyl)-benzo[1,2,5]thiadiazol-5-yl]-methanesulfonate.
Yield: 70 %
Melting point: 228-230 °C (ethyl acetate)
IR (KBr): 3096, 1517, 1322 cm⁻¹.
HNMR (CDCl₃, 125 MHz): 10.16 (s, 1H), 8.37 (s, 1H), 7.93 (s, 1H), 7.52 (s, 1H), 7.04 (m, 2H), 4.89 (s, 2H), 3.83 (s, 3H), 3.81 (bs, 6H)ppm.

Elemental analysis [calculated on the basis of the Formula C₁₆H₁₄N₄O₄S₂ (390.44)]

| | |
|---|---|
| Calculated: | C 49.22, H 3.61, N 14.35 % |
| Measured: | C 49.00, H 3.72, N 14.12 % |

### Example 11

### 9-(4-Fluorophenyl)-5H,7H-[1,2,5]tihadiazolo[3,4-h][2,3,4]benzothiadiazepin-6,6-dioxide

The title product is prepared according to the process B/1 starting from sodium [6-[(4-fluorophenyl)-carbonyl]-2,1,3-benzothiadiazol-5-yl]-methanesulfonate.
Yield: 68 %
Melting point: 222-225 °C (ethanol-DMF)
IR (KBr): 3137, 1597, 1569, 1508, 1326 cm⁻¹.
HNMR (DMSO-*d*₆, 500 MHz): 10.34 (s, 1H), 8.38 (s, 1H), 8.02 (s, 1H), 7.80 (dd, J = 8.7, 5.5 Hz, 2H), 7.36 (t, J = 8.9 Hz, 2H), 4.96 (s, 2H) ppm.
CNMR (DMSO-*d*₆, 125 MHz): 172.04, 164.33 (d, J = 250.0 Hz), 154.43, 152.96, 136.26, 133.11, 132.42 (d, J = 2.9 Hz), 132.19 (d, J = 8.7 Hz), 122.60, 121.47, 115.81 (d, J = 22.0 Hz), 55.10 ppm.

Elemental analysis [calculated on the basis of the Formula C₁₄H₉FN₄O₄S₂ (348.38)]

| | |
|---|---|
| Calculated: | C 48.27, H 2.60, N 16.08, S 18.41 % |
| Measured: | C 48.30, H 2.55, N 16.17, S 18.43 % |

### Example 12

### 7-Chloro-5-(4-fluoro-phenyl)-1,3-dihydro-2,3,4-benzothiadiazepin-2,2-dioxide

The title product is prepared according to the process B/1 starting from sodium [4-chloro-2-(4-fluoro-benzoyl)-phenyl]-methanesulfonate.
Yield: 68 %
Melting point: 195-198 °C (methanol)
IR (KBr): 3222, 1602, 1508, 1329, 1157 cm⁻¹.
HNMR (CDCl₃, 500 MHz): 7.70 (dd, J=5.3 Hz, 2H), 7.57 (dd, J=8.2, 2.1 Hz, 1H), 7.46 (d, J=8.2 Hz, 1H), 7.25 (d, J=2.2 Hz, 1H), 7.15 (t, J=8.6 Hz, 2H), 7.08 (bs, 1H), 4.39 (s, 2H) ppm.
CNMR (CDCl₃, 125 MHz): 171.2, 165.1 (d, J=254.4 Hz), 135.3, 135.0, 131.7 (d, J=8.8 Hz), 131.7, 131.0 (d, J=2.9 Hz), 130.7, 129.5, 129.2, 116.0 (d, J=22.0 Hz), 55.7 ppm.

Elemental analysis [calculated on the basis of the Formula **C₁₄H₁₀ClFN₂O₂S** (324.76)]

| | |
|---|---|
| Calculated: | C 51.78, H 3.10, N 8.63 % |
| Measured: | C 51.61, H 3.16, N 8.71 % |

### Example 13

### 7-Fluoro-5-(4-fluoro-phenyl)-1,3-dihydro-2,3,4-benzothiadiazepin-2,2-dioxide

The title product is prepared according to the process B/1 starting from, sodium [4-fluoro-2-(4-fluoro-benzoyl)-phenyl]-methanesulfonate.
Yield: 62 %
Melting point: 196-200 °C (ethanol-hexane)
IR (KBr): 3201, 1602, 1507, 1318, 1163 cm⁻¹.
HNMR (DMSO-*d*₆, 500 MHz): 10.33 (s, 1H), 7.68 (m, 3H), 7.49 (td, J=8.5, 2.7 Hz, 1H), 7.34 (t, J=8.8 Hz, 2H), 7.10 (dd, J=9.2, 2.7 Hz, 1H), 4.71 (s, 2H) ppm.
CNMR (DMSO-*d*₆, 125 MHz): 170.4 (d, J=1.5 Hz), 164.1 (d, J=249.5 Hz), 161.2 (d, J=246.6 Hz), 136.1 (d, J=7.8 Hz), 132.2 (d, J=2.9 Hz), 131.9 (d, J=8.8 Hz), 131.8 (d, J=8.8 Hz), 128.9 (d, J=3.4 Hz), 118.2 (d, J=22.0 Hz), 115.9 (d, J=23.4 Hz), 115.8 (d, J=22.0 Hz), 54.6 ppm.

Elemental analysis [calculated on the basis of the Formula C₁₄H₁₀F₂N₂O₂S (308.31)]

| | |
|---|---|
| Calculated: | C 54.54, H 3.27, S 10.40, N 9.09 %. |
| Measured: | C 54.89, H 3.23, S 10.28, N 9.16 %. |

### Example 14

### 7,8-Dichloro-5-(4-fluoro-phenyl)-1,3-dihydro-2,3,4-benzothiadiazepin-2,2-dioxide

The title product is prepared according to the process B/1 starting from sodium [4,5-dichloro-2-(4-fluoro-benzoyl)-phenyl]-methanesulfonate.
Yield: 72 %
Melting point: 196-200 °C (acetonitrile)
IR (KBr): 3443, 3200, 1599, 1508, 1327, 1166 cm⁻¹.
HNMR (DMSO-*d*₆, 500 MHz): 10.45 (s, 1H), 8.00 (s, 1H), 7.69 (dd, J=9.0, 5.5 Hz, 2H), 7.51 (s, 1H), 7.35 (t, J=8.8 Hz, 2H), 4.77 (s, 2H) ppm.
CNMR (DMSO-*d*₆, 125 MHz): 169.9, 164.2 (d, J=250 Hz), 134.5, 133.8, 133.0, 132.0 (d, J=8.8 Hz), 131.9 (d, J=2.9 Hz), 131.2, 131.1, 130.8 (d, J=22.0 Hz), 54.4 ppm.

Elemental analysis [calculated on the basis of the Formula C₁₄H₉Cl₂FN₂O₂S (359.21)]

| | |
|---|---|
| Calculated: | C 46.81, H 2.53, S 8.93, Cl 19.74, N 7.80 % |
| Measured: | C 46.55, H 2.63, S 8.78, Cl 19.51, N 7.71 % |

### Example 15

### 6-{[(4-Fluorophenyl)-carbonyl]-2,1,3-benzothiadiazol-5-yl}-methanesulfonic acid chloride

5.34 mmol sodium [6-[(4-fluorophenyl)-carbonyl]-2,1,3-benzothiadiazol-5-yl]-methanesulfonate is added to 5 ml thionyl chloride and are boiled for 2.5 hours. After cooling the reaction mixture is evaporated. The residue is dissolved in 10 ml chloroform and is clarified by carbon, after filtering the mixture is evaporated. The residue is triturated with hexane, filtered and dried.
Yield: 85 % Melting point: 148-151 °C (hexane)
IR (KBr): 2993, 1665, 1595, 1230, 850 cm⁻¹.
HNMR (CDCl₃, 500 MHz): 8.33 (s, 1H), 8.17 (s, 1H), 7.95 (dd, J = 5.4, 3.5 Hz, 2H), 7.21 (t, J = 8.5 Hz, 2H), 5.60 (s, 2H) ppm.
CNMR (CDCl₃, 125 MHz): 194.6, 166.10 (d, J = 256.8 Hz), 154.4, 153.6, 138.3, 133.5, 133.4, 132,7 (d, J = 3.0 Hz), 128.1, 127.0, 125.0, 115.9, 77.0 (t, J = 31.7 Hz), 66.7 ppm.

Elemental analysis [calculated on the basis of the Formula C₁₄H₈ClFN₂O₃S₂ (370.81)]

| | |
|---|---|
| Calculated: | C 45.35, H 2.17, N 7.55 % |
| Measured: | C 45.59, H 2.16, N 7.23 % |

### N(3)-alkylating reaction (Process "C")

119 mmol of 1,3-dihydro-2,3,4-benzothiadiazepin-2,2-dioxide, appropriately substituted on the benzene ring and containing an appropriately substituted phenyl group in position 5, is added to 1000 ml acetonitrile. 21.45 ml (144 mmol) of DBU and 146 mmol alkylating agent of the Formula (V) are added to the solution at room temperature. The reaction mixture is stirred at 20-60 °C until the starting material is used up. The amount of starting material is monitored by TLC.
The reaction mixture is evaporated and dissolved in 300 ml dichloromethane and 250 ml water. After separating, the aqueous layer is extracted with 100 ml dichloromethane. The combined organic phase is washed with 2×200 ml water, dried over MgSO₄ and evaporated.

Process C/1: the crystalline residue is triturated with 50 ml methanol and after stirring for 30 min, it is filtered and dried. An analytical sample is obtained by recrystallization from the solvent indicated.

Process C/2: If the residue is oily and not crystallized from methanol, the residue is subjected to flash chromatography on silica gel (20-fold amount) with hexane:ethyl acetate=1:1 eluent and it is crystallised. The solvent used for recrystallization is indicated right after the melting point of each compound.

Process C/3: if the residue is crystalline but soluble in methanol the residue is crystallized. The solvent used for recrystallization is indicated right after the melting point of each compound.

### Example 16

### 7-Chloro-5-(4-fluoro-phenyl)-3-methyl-1,3-dihydro-2,3,4-henzothiadiazepin-2,2-dioxide

The title product is prepared according to the process C/1 starting from, 7-chloro-5-(4-fluoro-phenyl)-1,3-dihydro-2,3,4-benzothiadiazepin-2,2-dioxide and methyl iodide.
Yield:.92 %
Melting point: 219-221 °C (acetonitrile)
IR (KBr): 2966, 2944, 1601, 1509, 1323, 1135 cm⁻¹.
HNMR (CDCl₃, 500 MHz): 7.71 (dd, J=9.0, 5.4 Hz, 2H), 7.55 (dd, J=8.3, 2.2 Hz, 1H), 7.45 (d, J=8.2 Hz, 1H), 7.24 (d, J=2.2 Hz, 1H), 7.14 (t, J=8.6 Hz, 2H), 4:35 (s, 2H), 3.49 (s, 3H) ppm.
CNMR (CDCl₃, 125 MHz): 169.9, 165.1 (d, J=253.9 Hz), 135.7, 134.9, 131.7 (d, J=8.8 Hz), 131.6, 130.9 (d, J=3.4 Hz), 130.4, 128.9, 128.7, 116.0 (d, J=22.0 Hz), 55.3, 35.3 ppm.

Elemental analysis [calculated on the basis of the Formula C₁₅H₁₂ClFN₂O₂S (338.79)

| | |
|---|---|
| Calculated: | C 53.18, H 3.57, N 8.27, S 9.46, Cl 10.46 % |
| Measured: | C 52.96, H 3.56, N 8.23, S 9.55, Cl 10.53 % |

### Example 17

### 7-Chloro-3-ethyl-5(4-fluoro-phenyl)-1,3-dihydro-2,3,4-benzothiadiazepin-2,2-dioxide

The title product is prepared according to the process C/1 starting from 7-chloro-5-(4-fluoro-phenyl)-1,3-dihydro-2,3,4-benzothiadiazepin-2,2-dioxide and ethyl iodide.
Yield: 90 %
Melting point: 171-172 °C (diisopropyl ether-ethanol)
IR (KBr): 2976, 1603, 1508, 1326, 1137 cm⁻¹.
HNMR (DMSO-*d*₆, 500 MHz): 7.72 (dd, J=8.3, 2.2 Hz, 1H), 7.71 (dd, J=8.9, 5.4 Hz, 2H), 7.70 (d, J=8.4 Hz, 1H), 7.36 (t, J=8.8 Hz, 2H), 7.31 (d, J=2.0 Hz, 1H), 4.84 (s, 2H), 3.45 (q, 2H), 1.05 (t, 3H) ppm.
CNMR (DMSO-*d*₆, 125 MHz): 170.5, 164.3 (d, J=250.0 Hz), 136.1, 133.1, 132.1 (d, J=8.8 Hz), 131.8 (d, J=2.9 Hz), 131.4, 131.4, 130.6, 128.1, 115.9 (d, J=22.0 Hz), 54.3, 42.4, 13.1 ppm.

Elemental analysis [calculated on the basis of the Formula C₁₆H₁₄ClFN₂O₂S (352.82)]

| | |
|---|---|
| Calculated: | C 54.47, H 4.00, Cl 10.05, S 9.09, N 7.94 % |
| Measured: | C 54.32, H 3.92, Cl 9.87, S 9.29, N 7.93 % |

### Example 18

### 7-Chloro-5-(4-fluoro-phenyl)-3-propyl-1,3-dihydro-2,3,4-benzothiadiazepin-2,2-dioxide

The title product is prepared according to the process C/2 starting from 7-chloro-5-(4-fluoro-phenyl)-1,3-dihydro-2,3,4-benzothiadiazepin-2,2-dioxide and propyl iodide.
Yield: 85 %
Melting point: 122-122.5 °C (diisopropyl ether-hexane)
IR (KBr): 2938, 1601, 1509, 1335, 1135 cm⁻¹.
HNMR (CDCl₃, 500 MHz): 7.72 (dd, J=8.9, 5.4 Hz, 2H), 7.55 (dd, J=8.2, 2.2 Hz, 1H), 7.45 (d, J=8.3 Hz, 1H), 7.22 (d, J=2.1 Hz, 1H), 7.14 (t, J=8.7 Hz, 2H), 4.34 (s, 2H), 3.53 (t, J=7.1 Hz, 2H), 1.49 (m, 2H), 0.72 (t, J=7.4 Hz, 3H) ppm.
CNMR (CDCl₃, 125 MHz): 170.9, 165.0 (d, J=254.4 Hz), 135.9, 134.6, 131.6 (d, J=9.3 Hz), 131.4, 131.0 (d, J=2.9 Hz), 130.4, 129.3, 128.2, 115.9 (d, J=22.0 Hz), 55.5, 49.2, 21.1, 10.8 ppm.

Elemental analysis [calculated on the basis of the Formula C₁₇H₁₆ClFN₂O₂S (366.84)]

| | |
|---|---|
| Calculated: | C 55.66, H 4.40, Cl 9.66, S 8.74, N 7.64 % |
| Measured: | C 55.68, H 4.41, Cl 9.62, S 8.82, N 7.57 % |

### Example 19

### 7-Chloro-5-(4-fluoro-phenyl)-3-(prop-2-inyl)-1,3-dihydro-2,3,4-benzothiadiazepin-2,2-dioxide

The title product is prepared according to the process C/2 starting from 7-chloro-5-(4-fluoro-phenyl)-1,3-dihydro-2,3,4-benzothiadiazepin-2,2-dioxide and 3-bromo-propine.
Yield: 91 %
Melting point: 139-140 °C (diisopropyl ether)
IR (KBr): 3305, 1605, 1508, 1329, 1131, 1065, 844 cm⁻¹.
HNMR (DMSO-*d*₆, 500 MHz): 7.76 (dd, J=8.3, 2.2 Hz, 1H), 7.72 (d, J=8.4 Hz, 1H), 7.71 (dd, J=8.9, 5.5 Hz, 2H), 7.39 (t, J=8.8 Hz, 2H), 7.32 (d, J=2.1 Hz, 1H), 4.95 (s, 2H), 4.21 (d, J=2.5 Hz, 2H), 3.25 (t, J=2.5 Hz, 1H ppm).
CNMR (DMSO-*d*₆, 125 MHz): 170.7, 164.5 (d, J=250.5 Hz), 135.9, 133.4, 132.3 (d, J=9.3 Hz), 131.7, 131.5, 131.5 (d, J=2.9 Hz), 130.4, 128.3, 116.0 (d, J=22.0 Hz), 78.3, 76.1, 54.3, 38.2 ppm.

Elemental analysis [calculated on the basis of the Formula C₁₇H₁₂ClFN₂O₂S (362.81)]

| | |
|---|---|
| Calculated: | C 56.28, H 3.33, Cl 9.77, S 8.84, N 7.72 % |
| Measured: | C 56.39, H 3.34, Cl 9.62, S 8.99, N 7.72 % |

### Example 20

### 7-Chloro-5-(4-fluoro-phenyl)-3-isopropyl-1,3-dihydro-2,3,4-benzothiadiazepin-2,2-dioxide

The title product is prepared according to the process C/1 starting from 7-chloro-5-(4-fluoro-phenyl)-1,3-dihydro-2,3,4-benzothiadiazepin-2,2-dioxide and isopropyl iodide.
Yield: 82 %
Melting point: 147-151 °C (diisopropyl ether)
IR (KBr): 3080, 1601, 1544, 1508, 1323, 1158, 1139, 852 cm⁻¹.
HNMR (CDCl₃, 500 MHz): 7.74 (dd, J=8.8, 5.4 Hz, 2H), 7.53 (dd, J=8.3, 2.2 Hz, 1H), 7.42 (d, J=8.2 Hz, 1H), 7.21 (d, J=2.1 Hz, 1H), 7.16 (t, J=8.5 Hz, 2H), 4.31 (s, 2H), 4.29 (m, 1H), 1.28 (d, J=6.6 Hz, 6H) ppm.
CNMR (CDCl₃, 125 MHz): 172.4, 165.0 (d, J=253.9 Hz), 135.8, 134.5, 131.6 (d, J=8.8 Hz), 131.4, 131.4 (d, J=3.4 Hz), 130.3, 129.8, 128.2, 115.9 (d, J=22.0 Hz), 56.4, 50.3, 20.7 ppm.

Elemental analysis [calculated on the basis of the Formula C₁₇H₁₆ClFN₂O₂S (366.84)]

| | |
|---|---|
| Calculated: | C 55.66, H 4.40, Cl 9.66, S 8.74, N 7.64 % |
| Measured: | C 55.33, H 4.41, Cl 9.55, S 8.60, N 7.54 % |

### Example 21

### 7-Chloro-5-(4-fluoro-phenyl)-3-(3-methyl-but-2-enyl)-1,3-dihydro-2,3,4-benzothiadiazepin-2,2-dioxide

The title product is prepared according to the process C/3 starting from 7-chloro-5-(4-fluoro-phenyl)-1,3-dihydro-2,3,4-benzothiadiazepin-2,2-dioxide and 1-bromo-3-methyl-but-2-ene.
Yield: 93 %
Melting point: 168-169 °C (diisopropyl ether)
IR (KBr): 2925, 1598, 1505, 1327, 1155, 1133 cm⁻¹
HNMR (CDCl₃, 500 MHz): 7.66 (dd, J=9.0, 5.4 Hz, 2H), 7.54 (dd, J=8.2, 2.1 Hz, 1H), 7.44 (d, J=8.2 Hz, 1H), 7.22 (d, J=2.2 Hz, 1H), 7.13 (t, J=8.4 Hz, 2H), 5.04 (t, J=1.3 Hz, 1H), 4.33 (s, 2H), 4.16 (d, J=6.9 Hz, 2H), 1.70 (d, J=1.1 Hz, 6H) ppm.
CNMR (CDCl₃, 125 MHz): 170.7, 164.9 (d, J = 253.9 Hz), 137.7, 135.9, 134.7, 131.6 (d, J = 8.8 Hz), 131.4, 131.2 (d, J = 3.4 Hz), 130.4, 129.2, 128.5, 117.9, 115.9 (d, J = 22.0 Hz), 55.7,45.88,25.8,18.1 ppm.

Elemental analysis [calculated on the basis of the Formula C₁₉H₁₈ClFN₂O₂S (392.88)]

| | |
|---|---|
| Calculated: | C 58.09, H 4.62, Cl 9.02, S 8.16, N 7.13 % |
| Measured: | C 58.01, H 4.68, Cl 8.96, S 8.15, N 7.07 % |

### Example 22

### 7-Chloro-5-(4-fluoro-phenyl)-3-(3-methyl-butyl)-1,3-dihydro-2,3,4-benzothiadiazepin-2,2-dioxide

The title product is prepared according to the process C/1 starting from 7-chloro-5-(4-fluoro-phenyl)-1,3-dihydro-2,3,4-benzothiadiazepin-2,2-dioxide and 1-bromo-3-methyl-but-2-ene.
Yield: 90 %
Melting point: 123-124 °C (diisopropyl ether-hexane)
IR (KBr): 2960, 603, 1556, 1510, 1326, 1134, 847 cm⁻¹.
HNMR (CDCl₃, 500 MHz): 7.72 (dd, 2H, J=8.9, 5.4 Hz), 7.54 (dd, 1H, J=8.2, 2.2 Hz), 7.44 (d, 1H, J=8.3 Hz), 7.23 (d, 1H, J=2.2 Hz), 7.15 (t, 2H, J=8.6 Hz), 4.33 (s, 2H), 3.59 (t, J=7.0 Hz, 2H), 1.39 (m, 1H), 1.34 (m, 2H), 0.74 (d, J=6.3 Hz, 6H) ppm.
CNMR (CDCl₃, 125 MHz): 170.9, 165.0 (d, J=254.4 Hz), 135.9, 134.6, 131.6 (d, J=8.8 Hz), 131.4, 131.0 (d, J=2.9 Hz), 130.4, 129.3, 128.2, 116.0 (d, J=22.0 Hz), 55.5, 46.2, 36.6, 25.7, 22.3 ppm.

Elemental analysis [calculated on the basis of the Formula C₁₉H₂₀ClFN₂O₂S (394.90)]

| | |
|---|---|
| Calculated: | C 57.79, H 5.10, Cl 8.98, S 8.12, N 7.09 % |
| Measured: | C 57.56, H 5.18, Cl 8.93, S 8.39, N 7.08 % |

### Example 23

### 3-Benzyl-7-chloro-5-(4-fluoro-phenyl)-1,3-dihydro-2,3,4-benzothiadiazepin-2,2-dioxide

The title product is prepared according to the process C/2 starting from 7-chloro-5-(4-fluoro-phenyl)-1,3-dihydro-2,3,4-benzothiadiazepin-2,2-dioxide and benzyl bromide.
Yield: 89 %
Melting point: 153 °C (diisopropyl ether)
IR (KBr): 1600, 1506, 1333, 1153, 842 cm⁻¹.
HNMR (CDCl₃, 500 MHz): 7.56 (dd, J=8.2, 2.2 Hz, 1H), 7.49 (dd, J=8.9, 5.4 Hz, 2H), 7.20 (m, 3H), 7.05 (t, J=8.6 Hz, 2H), 7.02 (d, J=2.0 Hz, 1H), 7.00 (d, J=2.0 Hz, 2H), 4.73 (s, 2H), 4.40 (s, 2H) ppm.
CNMR (CDCl₃, 125 MHz): 171.7, 165.0 (d, J=253.9 Hz), 136.0, 135.5, 134.7, 131.6 (d, J=9.3 Hz), 131.5, 130.8 (d, J=2.9 Hz), 130.4, 129.3, 128.5, 128.3, 128.2, 127.5, 115.8 (d, J=22.0 Hz), 55.7, 51.3 ppm.

Elemental analysis [calculated on the basis of the Formula C₂₁H₁₆ClFN₂O₂S (414.89)]

| | |
|---|---|
| Calculated: | C 60.80, H 3.89, Cl 8.55, S 7.73, N 6.75% |
| Measured: | C 60.73, H 3.75, Cl 8.42, S 7.88, N 6.75% |

### Example 24

### 7-Fluoro-5-(4-fluoro-phenyl)-3-methyl-1,3-dihydro-2,3,4-benzothiadiazepin-2,2-dioxide

The title product is prepared according to the process C/1 starting from 7-fluoro-5-(4-fluoro-phenyl)-1,3-dihydro-2,3,4-benzothiadiazepin-2,2-dioxide and methyl iodide.
Yield: 90 %
Melting point: 169-171 °C (ethanol)
IR (KBr): 2992, 2947, 1601, 1555, 1493, 1326, 1263, 1231 cm⁻¹.
HNMR (CDCl₃, 500 MHz): 7.72 (dd, J=8.4, 5.4 Hz, 2H), 7.50 (dd, J=8.4, 5.1 Hz, 1H), 7.28 (td, J=8.3, 2.3 Hz, 1H), 7.14 (t, J=8.3 Hz, 2H), 6.97 (dd, J=8.4, 2.4 Hz, 1H), 4.36 (s, 2H), 3.15 (s, 3H) ppm.
CNMR (CDCl₃, 125 MHz): 169.9, 165.0 (d, J=253.9 Hz), 162.0 (d, J=251.0 Hz), 136.1 (d, J=7.8 Hz), 131.7 (d, J=8.8 Hz), 131.1 (d, J=8.8 Hz), 130.9 (d, J=2.9 Hz), 126.5 (d, J=3.4 Hz), 118.6 (d, J=22.0 Hz), 115.9 (d, J=23.4 Hz), 115.9 (d, J=22.0 Hz), 55.1, 35.3 ppm.

Elemental analysis [calculated on the basis of the Formula **C₁₅H₁₂F₂N₂O₂S** (322.34)]

| | |
|---|---|
| Calculated: | C 55.89, H 3.75, S 9.95, N 8.69 % |
| Measured: | C 56.31, H 3.73, S 10.18, N 8.81 % |

### Example 25

### 7-Fluoro-3-ethyl-5-(4-fluoro-phenyl)-1,3-dihydro-2,3,4-benzothiadiazepin-2,2-dioxide

The title product is prepared according to the process C/1 starting from 7-fluoro-5-(4-fluoro-phenyl)-1,3-dihydro-2,3,4-benzothiadiazepin-2,2-dioxide and ethyl iodide.
Yield: 94 %
Melting point: 148-151 °C (diisopropyl-ether)
IR (KBr): 2977, 2935, 1602, 1553, 1507, 1330, 1233, 1144 cm⁻¹.
HNMR (DMSO-*d*₆, 500 MHz): 7.72 (m, 3H), 7.51 (td, J=8.6, 2.7 Hz, 1H), 7.36 (t, J=8.8 Hz, 2H), 7.14 (dd, J=9.0, 2.7 Hz, 1H), 4.80 (s, 2H), 3.44 (q, J=7.0 Hz, 2H), 1.04 (t, J=7.2 Hz, 3H) ppm.
CNMR (DMSO-*d*₆, 125 MHz): 170.6 (d, J=2.0 Hz), 164.3 (d, J=250.0 Hz), 161.4 (d, J=246.6 Hz), 136.2 (d, J=7.8 Hz), 132.1 (d, J=8.8 Hz), 131.8 (d, J=8.8 Hz), 131.8 (d, J=2.9 Hz), 128.0 (d, J=2.9 Hz), 118.5 (d, J=21.5 Hz), 115.9 (d, J=22.0 Hz), 115.5 (d, J=23.4 Hz), 54.1, 42.32, 13.1 ppm.

Elemental analysis [calculated on the basis of the Formula C₁₆H₁₄F₂N₂O₂S (336.36)]

| | |
|---|---|
| Calculated: | C 57.13, H 4.20, S 9.53, N 8.33 % |
| Measured: | C 56.86, H 4.25, S 9.59, N 8.33 % |

### Example 26

### 3-Butyl-7-fluoro-5-(4-fluoro-phenyl)-1,3-dihydro-2,3,4-benzothiadiazepin-2,2- dioxide

The title product is prepared according to the process C/1 starting from 7-fluoro-5-(4-fluoro-phenyl)-1,3-dihydro-2,3,4-benzothiadiazepin-2,2-dioxide and butyl iodide.
Yield: 88 %
Melting point: 99-103 °C (ethanol)
IR (KBr): 3072, 2971, 2938, 2909, 2861, 1602, 1549, 1497, 1331, 1233, 1156, 1131, 1080 cm⁻¹.
HNMR (DMSO-*d*₆, 500 MHz): 7.72 (m, 3H), 7.51 (td, J=8.7, 2.7 Hz, 1H), 7.36 (t, J=8.9 Hz, 2H), 7.24 (dd, J=9.0, 2.7 Hz, 1H), 4.81 (s, 2H), 3.41 (t, J=7.0 Hz, 2H), 1.40 (m, 2H), 1.05 (m, J=7.0 Hz, 2H), 0.74 (t, 3H) ppm.
CNMR (DMSO-*d*₆, 125 MHz): 170.8 (d, J=1.5 Hz), 164.3 (d, J=250.5 Hz), 161.3 (d, J=246.6 Hz), 136.3 (d, J=8.3 Hz), 132.0 (d, J=8.8 Hz), 131.7 (d, J=8.8 Hz), 131.7 (d, J=2.9 Hz), 128.2 (d, J=2.9 Hz), 118.5 (d, J=21.5 Hz), 115.9 (d, J=22.0 Hz), 115.3 (d, J=23.4 Hz), 54.1, 46.5, 29.4, 19.1, 13.5 ppm.

Elemental analysis [calculated on the basis of the Formula C₁₈H₁₉F₂N₂O₂S (364.42)]

| | |
|---|---|
| Calculated: | C 59.33, H 4.98, S 8.80, N 7.69 % |
| Measured: | C 59.06, H 5.04, S 8.99, N 7.61 % |

### Example 27

### 7-Fluoro-5-(4-fluoro-phenyl)-3-(3-methyl-but-2-enyl)-1,3-dihydro-2,3,4-benzothiadiazepin-2,2-dioxide

The title product is prepared according to the process C/1 starting from 7-fluoro-5-(4-fluoro-phenyl)-1,3-dihydro-2,3,4-benzothiadiazepin-2,2-dioxide and 1-bromo-3-methyl-but-2-ene.
Yield: 91 %
Melting point: 154-157 °C (ethanol)
IR (KBr): 2977, 2918, 1599, 1555, 1494, 1322, 1260, 1231, 1156, 1132 cm⁻¹.
HNMR (CDCl₃, 500 MHz): 7.67 (dd, J=8.8, 5.5 Hz, 2H), 7.48 (dd, J=8.5, 5.1 Hz, 1H), 7.27 (td, J=8.4, 2.6 Hz, 1H), 7.12 (t, J=8.8 Hz, 2H), 6.95 (dd, J=8.5, 2.7 Hz, 1H), 5.03 (t, J=7.1 Hz, 1H), 4.33 (s, 2H), 4.16 (d, J=7.1 Hz, 2H), 1.69 (s, 6H) ppm.
CNMR (CDCl₃, 125 MHz): 170.8 (d, J=1.5 Hz), 164.9 (d, J=253.9 Hz), 162.0 (d, J=250.5 Hz), 137.6, 136.2 (d, J=7.8 Hz), 131.6 (d, J=8.8 Hz), 131.2 (d, J=3.4 Hz), 131.0 (d, J=7.8 Hz), 126.8 (d, J=3.4 Hz), 118.5 (d, J=21.5 Hz), 117.9, 115.8 (d, J=22.0 Hz), 115.7 (d, J=23.0 Hz), 55.5, 45.8, 25.8, 18.1 ppm.

Elemental analysis [calculated on the basis of the Formula C₁₉H₁₈F₂N₂O₂S (376.43)]

| | |
|---|---|
| Calculated: | C 60.63, H 4.82, S 8.52, N 7.44 % |
| Measured: | C 60.48, H 4.93, S 8.42, N 7.40 % |

### Example 28

### 7-Fluoro-5-(4-fluoro-phenyl)-3-(3-methyl-butyl)-1,3-dihydro-2,3,4-benzothiadiazepin-2,2-dioxide

The title product is prepared according to the process C/1 starting from 7-fluoro-5-(4-fluoro-phenyl)-1,3-dihydro-2,3,4-benzothiadiazepin-2,2-dioxide and 1-bromo-3-methyl-but-2-ene.
Yield: 90 %
Melting point: 130-133 °C (ethanol)
IR (KBr): 2956, 2923, 2869, 1602, 1555, 1495, 1325, 1263, 1232, 1158, 1133, 1079 cm⁻¹.
HNMR (CDCl₃, 500 MHz): 7.73 (dd, J=8.9, 5.4 Hz, 2H), 7.49 (dd, J=8.5, 5.1 Hz, 1H), 7.28 (td, J=8.3, 2.7 Hz, 1H), 7.14 (t, J=8.7 Hz, 2H), 6.96 (dd, J=8.4, 2.7 Hz, 1H), 4.33 (s, 2H), 3.59 (t, J=7.2 Hz, 2H), 1.39 (m, 1H), 1.34 (q, J=6.8 Hz, 2H), 0.81 (d, J=6.4 Hz, 6H) ppm.
CNMR (CDCl₃, 125 MHz): 170.9 (d, J=1.5 Hz), 165.0 (d, J=254.4 Hz), 162.0 (d, J=251.0 Hz), 136.3 (d, J=7.3 Hz), 131.6 (d, J=8.8 Hz), 131.0 (d, J=8.3 Hz), 131.0 (d, J=3.9 Hz), 126.9 (d, J=2.3 Hz), 118.5 (d, J=22.0 Hz), 115.9 (d, J=22.0 Hz), 115.5 (d, J=23.4 Hz), 55.4, 46.1, 36.6, 25.7, 22.3 ppm.

Elemental analysis [calculated on the basis of the Formula C₁₉H₂₀F₂N₂O₂S (378.44)]

| | |
|---|---|
| Calculated: | C 60.30, H 5.33, S 8.47, N 7.40 % |
| Measured: | C 60.10, H 5.44, S 8.50, N 7.37 % |

### Example 29

### 7-Fluoro-5-(4-fluoro-phenyl)-3-hexyl-1,3-dihydro-2,3,4-benzothiadiazepin-2,2-dioxide

The title product is prepared according to the process C/2 starting from 7-fluoro-5-(4-fluoro-phenyl)-1,3-dihydro-2,3,4-benzothiadiazepin-2,2-dioxide and hexyl bromide.
Yield: 82 %
Melting point: 93-96 °C (IPA)
IR (KBr): 2930, 2858, 1600, 1554, 1495, 1326, 1263, 1231, 1158, 1076 cm⁻¹. HNMR (DMSO-*d*₆, 500 MHz): 7.73 (m, 3H), 7.51 (td, J=8.6, 2.7 Hz, 1H), 7.36 (t, J=8.8 Hz, 2H), 7.11 (dd, J=8.9, 2.7 Hz, 1H), 4.81 (s, 2H), 3.41 (t, J=6.4 Hz, 2H), 1.42 (m, 2H), 1.10 (m, 4H), 0.98 (m, 2H), 0.73 (t, J=7.0 Hz, 3H) ppm.
CNMR (DMSO-*d*₆, 125 MHz): 171.0 (d, J=1.5 Hz), 164.4 (d, J=250.5 Hz), 161.3 (d, J=247.1 Hz), 136.3 (d, J=7.8 Hz), 132.0 (d, J = 8.8 Hz), 131.7 (d, J=8.3 Hz), 131.6 (d, J=2.9 Hz), 128.2 (d, J=2.9 Hz), 118.4 (d, J=22.0 Hz), 115.9 (d, J=22.0 Hz), 115.3 (d, J=23.4 Hz), 54.2, 46.7, 30.7, 27.1, 25.4, 22.1, 13.8 ppm.

Elemental analysis [calculated on the basis of the Formula C₂₀H₂₂F₂N₂O₂S (392.47)]

| | |
|---|---|
| Calculated: | C 61.21, H 5.65, S 8.17, N 7.14 % |
| Measured: | C 61.15, H 5.83, S 8.13, N 7.10 % |

### Example 30

### 3-Benzyl-7-fluoro-5-(4-fluoro-phenyl)-1,3-dihydro-2,3,4-benzothiadiazepin-2,2-dioxide

The title product is prepared according to the process C/3 starting from 7-fluoro-5-(4-fluoro-phenyl)-1,3-dihydro-2,3,4-benzothiadiazepin-2,2-dioxide and benzyl bromide.
Yield: 90 %
Melting point: 184-187 °C (ethanol)
IR (KBr): 3430, 2995, 2949, 2926, 1598, 1547, 1494, 1320, 1232, 1153, 1129, 1074 cm⁻¹.
HNMR (DMSO-*d*₆, 500 MHz): 7.77 (dd, J=8.6, 5.4 Hz, 1H), 7.55 (td, J=8.7, 2.7 Hz, 1H), 7.53 (dd, J=9.0, 5.5 Hz, 2H), 7.30 (t, J=8.8 Hz, 2H), 7.24 (t, J=1.8 Hz, 2H), 7.21 (t, J=1.5 Hz, 1H), 7.00 (d, J=2.5 Hz, 2H), 6.99 (dd, J = 9.0, 2.7 Hz, 1H), 4.92 (s, 2H), 4.64 (s, 2H) ppm.
CNMR (DMSO-*d*₆, 125 MHz): 171.1 (d, J=1.5 Hz), 164.3 (d, J=250.0 Hz), 161.4 (d, J=247.6 Hz), 136.3 (d, J=7.8 Hz), 136.2, 132.0 (d, J=9.3 Hz), 131.9 (d, J=8.8 Hz), 131.4 (d, J=2.9 Hz), 128.4, 128.2 (d, J=2.0 Hz), 128.1, 127.4, 118.6 (d, J=22.0 Hz), 115.9 (d, J=22.0 Hz), 115.2 (d, J=23.4 Hz), 54.1, 50.8 ppm.

Elemental analysis [calculated on the basis of the Formula C₂₁H₁₆F₂N₂O₂S (398.44)]

| | |
|---|---|
| Calculated: | C 63.31, H 4.05, S 8.05, N 7.03 % |
| Measured: | C 63.03, H 4.03, S 7.91, N 6.98 % |

### Example 31

### 7-Fluoro-5-(4-fluoro-phenyl)-3-(3-phenyl-propyl)-1,3-dihydro-2,3,4-benzothiadiazepin-2,2-dioxide

The title product is prepared according to the process C/1 starting from 7-fluoro-5-(4-fluoro-phenyl)-1,3-dihydro-2,3,4-benzothiadiazepin-2,2-dioxide and 3-phenyl-propyl bromide.
Yield: 80 %
Melting point 163-167 °C (ethanol)
IR (KBr): 2992, 2939, 2867, 1601, 1557, 1495, 1451, 1424, 1329, 1262, 1230, 1158, 1059 cm⁻¹.
HNMR (DMSO-*d*₆, 500 MHz): 7.74 (m, 3H), 7.53 (td, J=8.7, 2.7 Hz, 1H), 7.37 (t, J=8.8 Hz, 2H), 7.22 (t, J=7.4 Hz, 2H), 7.20 (dd, J=8.9, 2.7 Hz, 1H), 7.14 (t, J=7.3 Hz, 1H), 7.02 (d, J=7.1 Hz, 2H), 4.84 (s, 2H), 3.43 (t, J=6.8 Hz, 2H), 2.31 (t, J=7.7 Hz, 2H), 1.72 (qn, J=7.8 Hz, 2H) ppm.
CNMR (DMSO-*d*₆, 125 MHz): 171.2 (d, J=1.5 Hz), 164.4 (d, J=250.0 Hz), 161.4 (d, J=247.1 Hz), 141.3, 136.3 (d, J=7.8 Hz), 132.1 (d, J=9.3 Hz), 131.8 (d, J=8.8 Hz), 131.6 (d, J=2.9 Hz), 128.4, 128.4, 128.2 (d, J=2.9 Hz), 125.9, 118.5 (d, J=21.5 Hz), 115.9 (d, J=22.0 Hz), 115.4 (d, J=23.9 Hz), 54.2, 46.2, 31.9, 29.4 ppm.

Elemental analysis [calculated on the basis of the Formula C₂₃H₂₀F₂N₂O₂S (426.49)]

| | |
|---|---|
| Calculated: | C 64.77, H 4.73, S 7.52, N 6.57 % |
| Measured: | C 64.70, H 4.89, S 7.58, N 6.56 % |

### Example 32

### 5-(3-Fluoro-phenyl)-7-chloro-3-methyl-1,3-dihydro-2,3,4-benzothiadiazepin-2,2-dioxide

The title product is prepared according to the process C/1 starting from 7-chloro-5-(3-ffuoro-phenyl)-1,3-dihydro-2,3,4-benzothiadiazepin-2,2-dioxide and methyl iodide.
Yield: 85 %
Melting point 172-173 °C (ethanol)
IR (KBr): 2992, 2939, 2867, 1601, 1557, 1495, 1451, 1424, 1329, 1262, 1230, 1158, 1059 cm⁻¹.
HNMR (CDCl₃, 500 MHz): 7.56 (dd, J=8.2, 2.2 Hz, 1H), 7.49 (m, 1H), 7.45 (d, J=8.2 Hz, 1H), 7.41 (m, 2H), 7.25 (m, 2H), 4.36 (s, 2H), 3.16 (s, 3H) ppm. CNMR (CDCl₃, 125 MHz): 169.7 (d, J=2.9 Hz), 162.8 (d, J=248.0 Hz), 136.8 (d, J=7.3 Hz), 135.4, 134.9, 131.7, 130.5, 130.3 (d, J=7.8 Hz), 128.9, 128.6, 125.5 (d, J=2.9 Hz), 118.9 (d, J=21.5 Hz), 116.0 (d, J=23.4 Hz), 55.3, 35.4.

Elemental analysis [calculated on the basis of the Formula C₁₅H₁₂ClFN₂O₂S (338.79)]

| | |
|---|---|
| Calculated: C | 53.18, H 3.57, Cl 10.46, S 9.46, N 8.27 % |
| Measured: | C 53.00, H 3.60, Cl 10.37, S 9.53, N 8.17 % |

### Example 33

### 8-Cyano-5-(4-fluoro-phenyl)-3-methyl-1,3-dihydro-2,3,4-benzothiadiazepin-2,2-dioxide

The title product is prepared according to the process C/1 starting from 7-chloro-5-(3-fluoro-phenyl)-1,3-dihydro-2,3,4-benzothiadiazepin-2,2-dioxide and methyl iodide.
Yield: 80 %
Melting point 284-285 °C (methanol)
HNMR (CDCl₃, 500 MHz): 7.81 (d, J=1.0 Hz, 1H), 7.78 (dd, J=8.1, 1.6 Hz, 1H), 7.69 (dd, J=8.9, 5.3 Hz, 1H), 7.15 (t, J=8.6 Hz, 2H), 4.41 (s, 2H), 3.15 (s, 3H) ppm.
CNMR (CDCl₃, 125 MHz): 169.7, 165.2 (d, J=254.9 Hz), 138.3, 132.4, 132.2, 131.8, 131.6 (d, J=9.3 Hz), 130.4 (d, J=2.9 Hz), 129.6, 117.1, 116.1 (d, J=22.0 Hz), 115.4, 55.2, 35.3 ppm.

Elemental analysis [calculated on the basis of the Formula C₁₆H₁₂FN₃O₂S (329.36)]

| | |
|---|---|
| Calculated: | C 58.35, H 3.67, S 9.74, N 12.76 % |
| Measured: | C 57.96, H 3.69, S 9.87, N 12.62 % |

### Example 34

### 8-Fluoro-5-(4-fluoro-phenyl)-3-methyl-1,3-dihydro-2,3,4-benzothiadiazepin-2,2-dioxide

The title product is prepared according to the process C/1 starting from 8-fluoro-5-(4-fluoro-phenyl)-1,3-dihydro-2,3,4-benzothiadiazepin-2,2-dioxide and methyl iodide.
Yield: 92 %
Melting point 205-206 °C (methanol)
HNMR (CDCl₃, 500 MHz): 7.70 (dd, J=8.4, 5.4 Hz, 2H), 7.25 (m, 2H), 7.18 (dt, J=8.3, 2.4 Hz, 1H), 7.12 (t, J=8.4 Hz, 2H), 4.35 (s, 2H), 3.14 (d, J=0.6 Hz, 3H) ppm.
CNMR (CDCl₃, 125 MHz): 170.3, 165.0 (d, J=253.4 Hz), 163.9 (d, J=253.9 Hz), 132.9 (d, J=8.8 Hz), 131.7 (d, J=8.8 Hz), 131.3 (d, J=3.4 Hz), 131.1 (d, J=9.3 Hz), 130.4 (d, J=3.4 Hz), 116.5 (d, J=22.9 Hz), 115.9 (d, J=22.0 Hz), 115.8 (d, J=22.0 Hz), 55.5 (d, J=2.0 Hz), 35.3 ppm.

Elemental analysis [calculated on the basis of the Formula C₁₅H₁₂F₂N₂O₂S (322.34)]

| | |
|---|---|
| Calculated: | C 55.89, H 3.75, S 9.95, N 8.69 % |
| Measured: | C 55.40, H 3.67, S 9.95, N 8.57 % |

### Example 35

### 5-(4-Fluoro-phenyl)-8-chloro-3-methyl-1,3-dihydro-2,3,4-benzothiadiazepin-2,2-dioxide

The title product is prepared according to the process C/1 starting from 8-chloro-5-(4-fluoro-phenyl)-1,3-dihydro-2,3,4-benzothiadiazepin-2,2-dioxide and methyl iodide.
Yield: 90 %
Melting point 250-251 °C (methanol)
HNMR (CDCl₃, 500 MHz): 7.70 (dd, J=8.9, 5.4 Hz, 2H), 7.52 (d, J=2.0 Hz, 1H), 7.46 (dd, J=8.3, 2.0 Hz, 1H), 7.20 (d, J=8.3 Hz, 1H), 7.13 (t, J=8.5 Hz, 2H) ppm. CNMR (CDCl₃, 125 MHz): 170.3, 165.0 (d, J=253.9 Hz), 137.7, 132.5, 132.1, 131.7 (d, J=9.3 Hz), 131.1 (d, J=3.4 Hz), 130.1, 129.3, 128.9, 115.9 (d, J=21.7 Hz), 55.4, 35.3 ppm.

Elemental analysis [calculated on the basis of the Formula C₁₅H₁₂ClFN₂O₂S (338.79)]

| | |
|---|---|
| Calculated: | C 53.18, H 3.57, Cl 10.46, S 9.46, N 8.27 % |
| Measured: | C 52.79, H 3.62, Cl 10.90, S 9.90, N 8.16 % |

### Example 36

### 5-(4-Fluoro-phenyl)-3-methyl-8-trifluoromethyl-1,3-dihydro-2,3,4-benzothiadiazepin-2,2-dioxide

The title product is prepared according to the process C/1 starting from 5-(4-fluoro-phenyl)-8-(trifluoromethyl)-1,3-dihydro-2,3,4-benzothiadiazepin-2,2-dioxide and methyl iodide.
Yield: 88 %
Melting point 160-162 °C (methanol)
HNMR (CDCl₃, 500 MHz): 7.78 (s, 1H), 7.75 (d, J=8.7 Hz, 1H), 7.71 (dd, J=8.7, 5.4, 2H), 7.41 (d, J=7.9 Hz, 1H), 7.14 (t, J=8.5 Hz, 2H), 4.44 (s, 2H), 3.16 (s, 3H) ppm.
CNMR (CDCl₃, 125 MHz): 170.0, 165.1 (d, J=254.4 Hz), 137.5, 133.4 (q, J=33.2 Hz), 131.7 (d, J=9.3 Hz), 131.4, 130.8 (d, J=3.4 Hz), 129.4, 126.0 (d, J=3.7 Hz), 125.6 (q, J=3.6 Hz), 123.2 (q, J=272.9 Hz), 116.0 (d, J=22.0 Hz), 55.6, 35.3 ppm.

Elemental analysis [calculated on the basis of the Formula C₁₆HnF₄N₂O₂S (372.34)]

| | |
|---|---|
| Calculated: | C 51.61, H 3.25, S 8.61, N 7.52 % |
| Measured: | C 51.52, H 3.32, S 8.54, N 7.46 % |

### Example 37

### 7,8-Dichloro-5-(4-fluoro-phenyl)-3-methyl-1,3-dihydro-2,3,4-benzothiadiazepin-2,2-dioxide

The title product is prepared according to the process C/1 starting from 8-dichloro-5-(4-fluoro-phenyl)-1,3-dihydro-2,3,4-benzothiadiazepin-2,2-dioxide and methyl iodide.
Yield: 92 %
Melting point 236-239 °C (IPA-DMF)
IR (KBr): 2970, 2921, 1601, 1546, 1509, 1475, 1328, 1229, 1159, 1136, 1071. HNMR (DMSO-*d*₆, 500 MHz): 8.03 (s, 1H), 7.72 (dd, J=8.9, 5.5 Hz, 2H), 7.54 (s, 1H), 7.36 (t, J=8.9 Hz, 2H), 4.90 (s, 2H), 3.01 (s, 3H) ppm.
CNMR (DMSO-*d*₆, 125 MHz): 169.1, 164.4 (d, J=250.5 Hz), 134.4, 134.1, 132.3 (d, J=8.8 Hz), 132.0, 131.4, 131.3 (d, J=2.9 Hz), 131.2, 130.49, 115.9 (d, J=22.0 Hz), 53.5, 34.9 ppm.

Elemental analysis [calculated on the basis of the Formula C₁₆H₁₂F₄N₂O₂S (372.34)]

| | |
|---|---|
| Calculated: | C 48.27, H 2.97, Cl 19.00, S 8.59, N 7.51 % |
| Measured: | C 48.31, H 2.99, Cl 18.96, S 8.60, N 7.53 % |

### Example 38

### 9-(4-fluorophenyl)-7-methyl-5H,7H-[1,2,5]thiadiazolo[3,4-h] [2,3,4]benzothiadiazepin-6,6-dioxide

The title product is prepared according to the process C/1 starting from 9-(4-fluorophenyl)-5*H*,7*H*-[1,2,5]thiadiazolo[3,4-*h*][2,3,4]benzothiadiazepin-6,6-dioxide and methyl iodide.
Yield: 95 %
Melting point 272-275 °C (ethanol-DMF)
IR (KBr): 3137, 1597, 1569, 1508, 1326 cm⁻¹.
HNMR (DMSO-*d*₆, 500 MHz): 10.34 (s, 1H), 8.38 (s, 1H), 8.02 (s, 1H), 7.80 (dd, J = 8.7, 5.5 Hz, 2H), 7.36 (t, J = 8.9 Hz, 2H), 4.96 (s, 2H) ppm.
CNMR (DMSO-*d*₆, 125 MHz): 172.0, 164.3 (d, J = 250.0 Hz), 154.4, 153.0, 136.3, 133.1, 132.4 (d, J = 2.9 Hz), 132.2 (d, J = 8.7 Hz), 122.6, 121.5, 115.8 (d, J = 22.0 Hz), 55.1 ppm.

Elemental analysis [calculated on the basis of the Formula C₁₅H₁₁FN₄O₂S₂ (362.41)]

| | |
|---|---|
| Calculated: | C 49.71, H 3.06, N 15.46, S 17.70 % |
| Measured: | C 50.04, H 3.06, N 15.82, S 17.50 % |

### Example 39

### 7,8-Dichloro-3-ethyl-5-(4-fluoro-phenyl)-1,3-dihydro-2,3,4-benzothiadiazepin-2,2-dioxide

The title product is prepared according to the process C/3 starting from 7,8-dichloro-5-(4-fluoro-phenyl)-1,3-dihydro-2,3,4-benzothiadiazepin-2,2-dioxide and ethyl iodide.
Yield: 76 %
Melting point 218-221 °C (acetonitryle)
IR (KBr): 2986, 1601, 1326, 1138 cm⁻¹.
HNMR (DMSO-*d*₆, 500 MHz): 8.03 (s, 1H), 7.74 (dd, J=9.0, 5.5 Hz, 2H), 7.55 (s, 1H), 7.36 (t, J=8.8 Hz, 2H), 4.86 (s, 2H), 3.46 (q, 2H), 1.05 (t, 3H) ppm.
CNMR (DMSO-*d*₆, 125 MHz): 170.07, 164.43 (d, J = 250.5 Hz), 134.60, 134.10, 132.22 (d, J = 9.3 Hz), 132.21, 131.57 (d, J = 2.9 Hz), 131.44, 131.17, 130.29, 115.92 (d, J = 22.0 Hz), 53.81, 42.46, 13.08 ppm.

Elemental analysis [calculated on the basis of the Formula C₁₆H₁₃Cl₂FN₂O₂S (387.26)]

| | |
|---|---|
| Calculated: | C 49.62, H 3.38, S 8.28, Cl 18.31, N 7.23 % |
| Measured: C | 49.60, H 3.45, S 8.19, Cl 18.03, N 7.26 % |

### C(1)-alkylating reaction (Process "D")

15 mmol of 1,3-dihydro-2,3,4-benzothiadiazepine-2,2-dioxide, alkylated (aralkylated) in position 3, appropriately substituted on the aromatic carbocycle and containing an appropriately substituted phenyl group in position 5 is added to 100 ml tetrahydrofurane. To the solution is added 7 ml (17.5 mmol) 2.5 M BuLi in hexane under stirring in an acetone-dry ice bath at -78 °C.

After stirring for one hour at this temperature, 16.5 mmol alkyl iodide is added to the red solution in 8 ml tetrahydrofurane. After warming to room temperature, the reaction mixture is added to 150 ml ice and stirred half an hour. After extracting the aqueous mixture with 180 ml ethyl acetate, the organic layer is extracted with brine, dried over MgSO₄ and evaporated. The product is crystallized. The solvent used by crystallization is indicated at the melting points of each compound.

### Example 40

### 7-Chloro-5-(4-fluoro-phenyl)-1,3-dimethyl-1,3-dihydro-2,3,4-benzothiadiazepin-2,2-dioxide

The title product is prepared according to the process D starting from 7-chloro-5-(4-fluoro-phenyl)-3-methyl-1,3-dihydro-2,3,4-benzothiadiazepin-2,2-dioxide and methyl iodide.
Yield: 87 %
Melting point: 139-143 °C (2-propanol)
IR (KBr): 2937, 1602, 1550, 1508, 1317, 1236, 1143, 1041 cm⁻¹.
HNMR (CDCl₃, 500 MHz): 7.73 (dd, J=9.0, 5.4 Hz, 2H), 7.58 (dd, J=8.4, 2.2 Hz, 1H), 7.45 (d, J=8.4 Hz, 1H), 7.20 (d, J=2.2 Hz, 1H), 7.14 (t, J=9.0 Hz, 2H), 4.36 (q, J=7.1 Hz, 1H), 3.16 (s, 3H), 1.88 (d, J=7.1 Hz, 3H) ppm.
CNMR (CDCl₃, 125 MHz): 169.6, 165.0 (d, J=253.9 Hz), 136.1, 134.4, 133.4, 131.7 (d, J=8.8 Hz), 131.3, 130.9 (d, J=3.4 Hz), 128.4, 127.1, 115.9 (d, J=22.0 Hz), 57.4, 35.9, 10.4 ppm.

Elemental analysis [calculated on the basis of the Formula C₁₆H₁₄ClFN₂O₂S (352.82)]

| | |
|---|---|
| Calculated: | C 54.47, H 4.00, Cl 10.05, S 9.09, N 7.94 % |
| Measured: | C 54.56, H 4.09, Cl 9.90, S 9.00, N 7.93 % |

### Example 41

### 7-Chloro-1-ethyl-5-(4-fluoro-phenyl)-3-methyl-1,3-dihydro-2,3,4-benzothiadiazepin-2,2-dioxide

The title product is prepared according to the process D starting from 7-chloro-5-(4-fluoro-phenyl)-3-methyl-1,3-dihydro-2,3,4-benzothiadiazepin-2,2-dioxide and ethyl iodide.
Yield: 92 %
Melting point: 175-177 °C (IPA)
IR (KBr): 2970, 2935, 1603, 1509, 1316, 1146, 841 cm⁻¹.
HNMR (CDCl₃, 500 MHz): 7.80-7.70 (m, 2H), 7.6-7.4 (m, 2H), 7.21 (d, J=2.2 Hz, 1H), 7.14 (t, J=8.4 Hz, 2H), 4.16 (q, J=4.5 Hz, 1H), 3.14 (bs, 3H), 2.52-2.35 (m, 1H), 2.34-2.10 (m, 1H). 1.06 (t, J=7.3 Hz, 3H) ppm.
CNMR (CDCl₃, 125 MHz): 169.4 (d, J=102.5 Hz), 162.3, 136.0, 134.1, 132.4, 131.4 (d, J=9.1 Hz), 131.0 (d, J=12.5 Hz); 128.5, 128.2, 115.8 (d, J=21.9 Hz), 77.0 (t, J=32.0 Hz), 65.0, 35.6, 11.8 ppm.

Elemental analysis [calculated on the basis of the Formula C₁₇H₁₆ClFN₂O₂S (366.84)]

| | |
|---|---|
| Calculated: | C 55.66, H 4.40, Cl 9.66, S 8.74, N 7.64 % |
| Measured: | C 55.40, H 4.39, Cl 9.50, S 8.66, N 7.63 % |

## Claims

1. Compounds of the general Formula 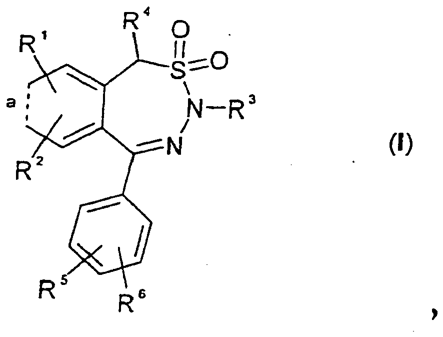 wherein
'a' represents single or double bond,
R¹ and R² represent, independently, hydrogen, halogen, cyano group, C₁₋₆ alkyl group, C₁₋₆ alkoxy group trifluoromethyl group or other C₁₋₆ alkyl group substituted by one or more halogen atom
or if
'a' represents a single bond,
R¹ and R² together form a group of the formula =N-S-N= attached to the atoms of the bond 'a' according to the partial Formula (A), 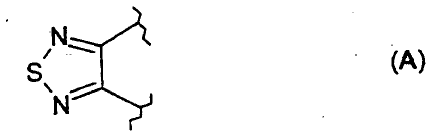
R³ and R⁴ represent, independently, hydrogen, C₁₋₆ alkyl group, C₁₋₆ alkenyl group, C₃₋₆ cycloalkyl group, C₁₋₆ aryl group or C₁₋₆ aralkyl group,
R⁵ and R⁶ represent, independently, hydrogen, halogen, cyano group, C₁₋₆ alkyl group, C₁₋₆ alkoxy group, trifluoromethyl group or other C₁₋₆ alkyl group substituted by one or more halogen atom
enantiomers and pharmaceutically suitable acid addition salts thereof.

2. A compound according to claim 1, selected from
8-cyano-5-(4-fluoro-phenyl)-1,3-dihydro-2,3,4-benzothiadiazepine-2,2-dioxide;
7-chloro-5-(3-fluoro-phenyl)-1,3-dihydro-2,3,4-benzothiadiazepine-2,2-dioxide,
9-(4-methyl-phenyl)-*5H*,*7H*-[1,2,5]thiadiazolo[3,4-*h*]-2,3,4-benzothiadiazepine-6,6-dioxide;
9-(4-methoxy-phenyl)-*5H*,*7H*-[1,2,5]thiadiazolo[3,4-*h*][2,3,4]benzothiadiazepine-6,6-dioxide;
9-(3,4-dimethoxy-phenyl)-*5H*,*7H*[1,2,5]thiadiazolo[3,4-*h*][2,3,4]benzothiadiazepine-6,6-dioxide;
9-(4-fluoro-phenyl]-*5H,7H-*(1,2,5)thiadiazolo[3,4-*h*][2,3,4]benzothiadiazepine-6,6-dioxide;
7-chloro-5-(4-fluoro-phenyl)-1,3-dihydro-2,3,4-benzothiadiazepien-2,2-dioxide;
7-fluoro-5-(4-fluoro-phenyl)-1,3-dihydro-2,3,4-benzothiadiazepine-2,2-dioxide;
7,8-dichloro-5-(4-fluoro-phenyl)-1,3-dihydro-2,3,4-benzothiadiazepine-2-dioxide;
7-chloro-5-(4-fluoro-phenyl)-3-methyl-1,3-dihydro-2,3,4-benzothiadiazepine-2,2-dioxide;
7-chloro-3-ethyl-5-(4-fluoro-phenyl)-1,3-dihydro-2,3,4-benzothiadiazepine-2,2-dioxide;
7-chloro-5-(4-fluoro-phenyl)-3-propyl-1,3-dihydro-2,3,4-benzothiadiazepine-2,2-dioxide;
7-chloro-5-(4-fluoro-phenyl)-3-(prop-2-inyl)-1,3-dihydro-2,3,4-benzothiadiazepine-2,2-dioxide;
7-chloro-5-(4-fluoro-phenyl)-3-isopropyl-1,3-dihydro-2,3,4-benzothiadiazepine-2,2-dioxide;
7-chloro-5-(4-fluoro-phenyl)-3-(3-methyl-but-2-enyl)-1,3-dihydro-2,3 ,4-benzothiadiazepine-2,2-dioxide;
7-chloro-5-(4-fluoro-phenyl)-3-(3-methyl-butyl)-1,3-dihydro-2,3,4-benzothiadiazepine-2,2-dioxide;
3-benzyl-7-chloro-5-(4-fluoro-phenyl)-1,3-dihydro-2,3,4-benzothiadiazepine-2,2-dioxide;
7-fluoro-5-(4-fluorf-phenyl)-3-methyl-1,3-dihydro-2,3,4-benzothiadiazepine-2,2-dioxide;
7-fluoro-3-ethyl-5-(4-fluoro-phenyl)-1,3-dihydro-2,3,4-benzothiadiazepine-2,2-dioxide;
3-butyl-7-fluoro-5-(4-fluoro-phenyl)-1,3-dihydro-2,3,4-benzothiadiazepine-2,2- dioxide;
7-fluoro-5-(4-fluoro-phenyl)-3-(3-methyl-but-2-enyl)-1,3-dihydro-2,3,4-benzothiadiazepine-2,2-dioxide;
7-fluoro-5-(4-fluoro-phenyl)-3-(3-methyl-butyl)-1,3-dihydro-2,3,4-benzothiadiazepine-2,2-dioxide;
7-fluoro-5-(4-fluoro-phenyl)-3-hexyl-1,3-dihydro-2,3,4-benzothiadiazepine-2,2-dioxide;
3-benzyl-7-fluoro-5-(4-fluoro-phenyl)-1,3-dihydro-2,3,4-benzothiadiazepine-2,2-dioxide;
7-fluoro-5-(4-fluoro-phenyl)-3-(3-phenyl-propyl)-1,3-dihydro-2,3,4-benzothiadiazepine-2,2-dioxide;
5-(3-fluoro-phenyl)-7-chloro-3-methyl-1,3-dihydro-2,3,4-benzothiadiazepine-2,2-dioxide;
8-cyano-5-(4-fluoro-phenyl)-3-methyl-1,3-dihydro-2,3,4-benzothiadiazepine-2,2-dioxide;
8-fluoro-5-(4-fluoro-phenyl)-3-methyl-1,3-dihydro-2,3,4-benzothiadiazepine-2,2-dioxide;
5-(4-fluoro-phenyl)-8-chloro-3-methyl-1,3-dihydro-2,3,4-benzothiadiazepine-2,2-dioxide;
5-(4-fluoro-phenyl)-3-methyl-8-trifluormethyl-1,3-dihydro-2,3,4-benzothiadiazepine-2,2-dioxide;
7,8-dichloro-5-(4-fluoro-phenyl)-3-methyl-1,3-dihydro-2,3,4-benzothiadiazepine-2,2-dioxide;
9-(4-fluoro-phenyl)-7-methyl-*5H*,*7H*-[1,2,5]thiadiazolo[3,4-*h*][2,3,4] benzothiadiazepine-6,6-dioxide;
7,8-dichloro-3-ethyl-5-(4-fluoro-phenyl)-1,3-dihydro-2,3,4-benzothiadiazepine-2,2-dioxide;
7-chloro-5-(4-fluoro-phenyl)-1,3-dimethyl-1,3-dihydro-2,3,4-benzothiadiazepine-2,2-dioxide;
7-chloro-1-ethyl-5-(4-fluoro-phenyl)-3-methyl-1,3-dihydro-2,3,4-benzothiadiazepine-2,2-dioxide
and/or enantiomers and/or pharmaceutically acceptable acid addition salts thereof.

3. Process for the preparation of the compounds of the general Formula (I), according to claim 1,
wherein R³ and R⁴ are hydrogen and R¹, R², R⁵ and R⁶ are defined in claim 1 and pharmaceutically suitable acid addition salts thereof, by reacting the compound of the Formula (II) 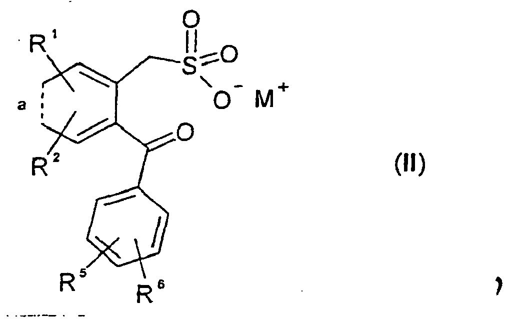 wherein R¹, R², R⁵ and R⁶ are defined in claim
1 and M represents monovalent or multivalent metals, with tionyl chloride and by subsequent reaction of the obtained compound of the general Formula (III) 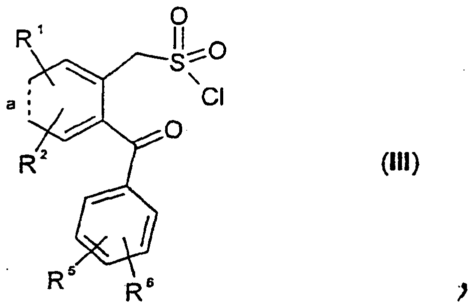 wherein R¹, R², R⁵ and R⁶ are defined in claim 1, with hydrazine.

4. Process according to claim 3 for the preparation of the compounds of the general Formula (I), according to claim 1,
wherein R⁴ is hydrogen and R¹ R², R⁵, R⁶ and R³ are defined in claim 1 provided that R³ is different from hydrogen, and pharmaceutically suitable acid addition salts thereof, by reacting the 2,3,4-benzothiadiazepine-dioxide derivatives of the general Formula (I) wherein R³ and R⁴ are hydrogen, R¹, R², R⁵ and R⁶ are defined in claim 1, with agents suitable for introducing an alkyl group, alkenyl group or aralkyl group, in the presence of a base.

5. Process according to claim 3 for the preparation of the compounds of the general Formula (I), according to claim 1,
wherein R⁴ is hydrogen and R¹, R², R⁵, R⁶ and R³ are defined in claim 1 provided that R³ is different from hydrogen, and pharmaceutically suitable acid addition salts thereof, by reacting compounds of the general formula (III) 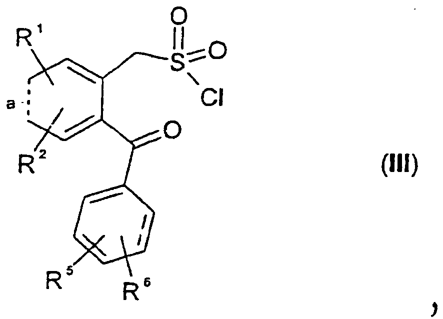 wherein R¹, R², R⁵ and R⁶ are defined in claim 1, with a substituted hydrazine of general formula (VI).
R³-NH-NH₂ (VI)
wherein R³ is defined in claim 1.

6. Process according to claim 3 for the preparation of the compounds of the general Formula (I), according to claim 1,
wherein R³ and R⁴ are defined in claim 1 provided that R³ and R⁴ are identical and different from hydrogen, and R¹, R², R⁵ and R⁶ are defined in claim 1, and pharmaceutically suitable acid addition salts thereof, by reacting the 2,3,4-benzothiadiazepine-dioxide derivatives of the general Formula (I), wherein R³ and R⁴ are hydrogen, R¹, R², R⁵ and R6 are defined in claim 1, with agents suitable for introducing an alkyl group, alkenyl group or aralkyl group, in the presence of a base.

7. Process according to claim 3 for the preparation of the compounds of the general Formula (I), of claim 1
wherein R³ and R⁴ are defined in claim 1 provided that R³ and R⁴ are different from hydrogen and R¹, R², R⁵ and R⁶ are defined in claim 1 and pharmaceutically suitable acid addition salts thereof, by reacting the 2,3,4-benzothiadiazepine-dioxide derivatives of the general Formula (I) wherein R⁴ is hydrogen, R¹, R², R⁵, R⁶ and R³ are defined in claim 1 provided that R³ is different from hydrogen, with agents suitable for introducing an alkyl group, alkenyl group or aralkyl group, in the presence of a base.

8. The compounds of general Formula (III) 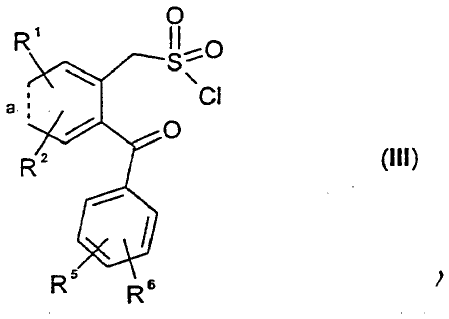 wherein R¹, R², R⁵ and R⁶ are defined in claim 1.

9. Process for the preparation of the compounds of general Formula (III) 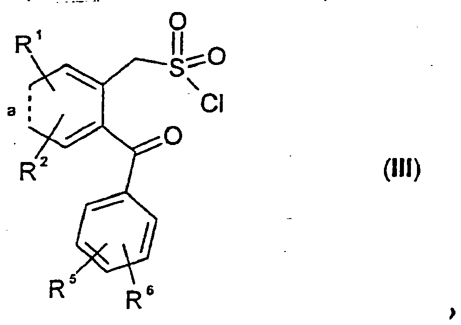 wherein R¹, R², R⁵ and R⁶ are defined in claim 1 which comprises reacting the compound of general Formula (II) 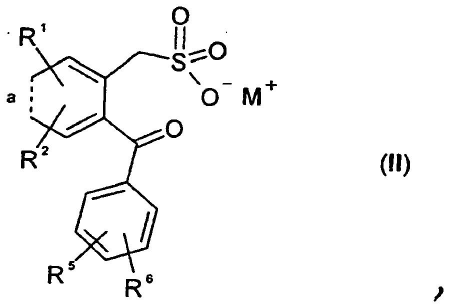 wherein R¹, R², R⁵ and R⁶ are defined in claim 1 with thionyl chloride.

10. The compounds of general Formula (II) 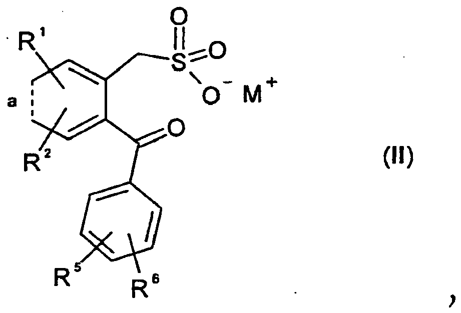 wherein R¹, R², R⁵ and R⁶ are
defined in claim 1, M represents metal atom, preferably alkali metal atom, more preferably sodium atom.

11. Compounds as defined in claim 10
sodium [4-chloro-2-(4-fluoro-benzoyl)-phenyl]-methanesulfonate;
sodium [6-[(3,4-dimethoxy-phenyl)-carbonyl]-2,1,3-benzothiadiazole-5-yl]-methanesulfonate;
sodium [6-[(4-fluoro-phenyl)-carbonyl]-2,1,3-benzothiadiazole-5-yl]-methanesulfonate;
sodium-[6-[(4-methoxy-phenyl)-carbonyl]-2,1,3-benzothiadiazole-5-yl]-methanesulfonate.

12. Process for the preparation of the derivatives of general Formula (II) 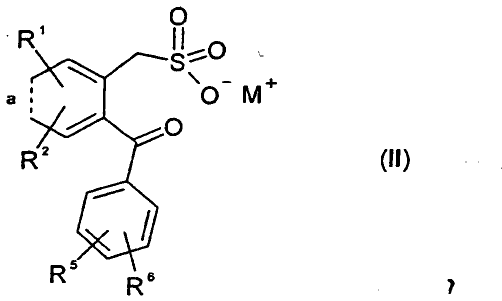 wherein
R¹, R², R⁵ and R⁶ are defined in claim 1, which comprises reacting the
compound of general Formula (IV) 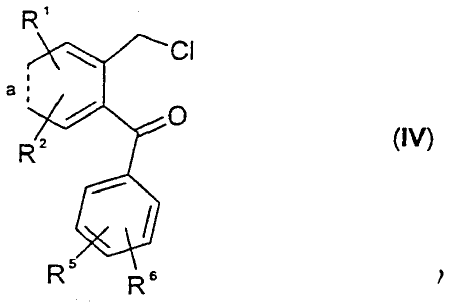 wherein R¹, R², R⁵ and R⁶ are defined in
claim 1 with ammonium sulfite or alkali sulfite in the present of water or water-immiscible solvent, preferably in acetone, dioxane or in an aliphatic alcohol comprising 1 to 4 carbon atoms or in the mixture thereof.

13. Compounds of the general Formula (I) as defined in claim 1 for the use as a medicament.

14. Pharmaceutical compositions comprising as active ingredient one or more compounds of general Formula (I) as defined in claims 1 and/or enantiomers and/or pharmaceutically suitable acid addition salts thereof in a therapeutically effective dose together with one or more pharmaceutically applicable diluent, excipient and/or inert carrier.

15. Use of the pharmaceutical composition as defined in claim 14 for the preparation of medicaments for the treatment and/or prevention of diseases of the central nervous system.

16. Compounds of the general Formula (I) as defined in claim 1 for the use as a medicament in the treatment and/or prevention of diseases of the central nervous system.

17. Use of one or more compounds of general Formula (I) as defined in claim 1 and/or enantiomers and/or pharmaceutically suitable acid additional salts thereof for the preparation of a medicament for the treatment of central nervous system disorders.

## Patentansprüche

1. Verbindungen mit der allgemeinen Formel (I) 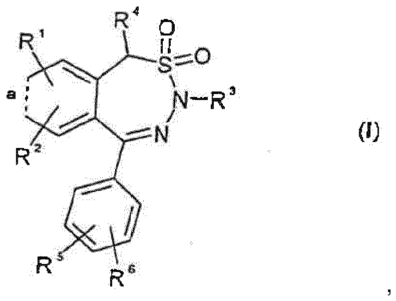 wobei
a eine Einfach- oder eine Doppelbindung darstellt,
R¹ und R² unabhängig voneinander Wasserstoff, Halogen, eine Cyanogruppe, eine C₁₋₆-Alkylgruppe, eine C₁₋₆-Alkoxygruppe, eine Trifluormethylgruppe oder eine andere durch ein oder mehrere Halogenatome substituierte C₁₋₆-Alkylgruppe darstellen,
oder falls
a eine Einfachbindung darstellt,
R¹ und R² gemeinsam eine Gruppe mit der Formel =N-S-N= bilden, die an die Atome der Bindung a gemäß der Teilformel (A) 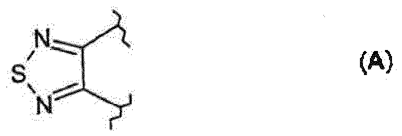 gebunden ist,
R³ und R⁴ unabhängig voneinander Wasserstoff, eine C₁₋₆-Alkylgruppe, eine C₁₋₆-Alkenylgruppe, eine C₃₋₆-Cycloalkylgruppe, eine C₁₋₆-Arylgruppe oder eine C₁₋₆-Ararylgruppe darstellen,
R⁵ und R⁶ unabhängig voneinander Wasserstoff, Halogen, eine Cyanogruppe, eine C₁₋₆-Alkylgruppe, eine C₁₋₆-Alkoxygruppe, eine Trifluormethylgruppe oder
eine andere durch ein oder mehrere Halogenatome substituierte C₁₋₆-Alkylgruppe darstellen,
Enantiomere und deren pharmazeutisch geeignete Säureadditionssalze.

2. Verbindung nach Anspruch 1, ausgewählt aus 8-Cyano-5-(4-fluorphenyl)-1,3-dihydro-2,3,4-benzothiadiazepin-2,2-dioxid;
7-Chlor-5-(3-fluorphenyl)-1,3-dihydro-2,3,4-benzothiadiazepin-2,2-dioxid;
9-(4-Methylphenyl)-5H,7H-[1,2,5]thiadiazol[3,4-h]-2,3,4-benzothiadiazepin-6,6-dioxid;
9-(4-Methoxyphenyl)-5H,7H-[1,2,5]thiadiazol[3,4-h][2,3,4]benzothiadiazepin-6,6-dioxid;
9-(3,4-Dimethoxyphenyl)-5H,7H-[1,2,5]thiadiazol[3,4-h][2,3,4]benzothiadiazepin-6,6-dioxid;
9-(4-Fluorphenyl)-5H,7H-[1,2,5]thiadiazol[3,4-h][2,3,4]benzothiadiazepin-6,6-dioxid;
7-Chlor-5-(4-fluorphenyl)-1,3-dihydro-2,3,4-benzothiadiazepin-2,2-dioxid;
7-Fluor-5-(4-fluorphenyl)-1,3-dihydro-2,3,4-benzothiadiazepin-2,2-dioxid;
7,8-Dichlor-5-(4-fluorphenyl)-1,3-dihydro-2,3,4-benzothiadiazepin-2,2-dioxid;
7-Chlor-5-(4-fluorphenyl)-3-methyl-1,3-dihydro-2,3,4-benzothiadiazepin-2,2-dioxid;
7-Chlor-3-ethyl-5-(4-fluorphenyl)-1,3-dihydro-2,3,4-benzothiadiazepin-2,2-dioxid;
7-Chlor-5-(4-fluorphenyl)-3-propyl-1,3-dihydro-2,3,4-benzothiadiazepin-2,2-dioxid;
7-Chlor-5-(4-fluorphenyl)-3-(prop-2-inyl)-1,3-dihydro-2,3,4-benzothiadiazepin-2,2-dioxid;
7-Chlor-5-(4-fluorphenyl)-3-isopropyl-1,3-dihydro-2,3,4-benzothiadiazepin-2,2-dioxid;
7-Chlor-5-(4-fluorphenyl)-3-(3-methylbut-2-enyl)-1,3-dihydro-2,3,4-benzothiadiazepin-2,2-dioxid;
7-Chlor-5-(4-fluorphenyl)-3-(3-methylbutyl)-1,3-dihydro-2,3,4-benzothiadiazepin-2,2-dioxid;
3-Benzyl-7-chlor-5-(4-fluorphenyl)-1,3-dihydro-2,3,4-benzothiadiazepin-2,2-dioxid;
7-Fluor-5-(4-fluorphenyl)-3-methyl-1,3-dihydro-2,3,4-benzothiadiazepin-2,2-dioxid;
7-Fluor-3-ethyl-5-(4-fluorphenyl)-1,3-dihydro-2,3,4-benzothiadiazepin-2,2-dioxid;
3-Butyl-7-fluor-5-(4-fluorphenyl)-1,3-dihydro-2,3,4-benzothiadiazepin-2,2-dioxid;
7-Fluor-5-(4-fluorphenyl)-3-(3-methylbut-2-enyl)-1,3-dihydro-2,3,4-benzothiadiazepin-2,2-dioxid;
7-Fluor-5-(4-fluorphenyl)-3-(3-methylbutyl)-1,3-dihydro-2,3,4-benzothiadiazepin-2,2-dioxid;
7-Fluor-5-(4-fluorphenyl)-3-hexyl-1,3-dihydro-2,3,4-benzothiadiazepin-2,2-dioxid;
3-Benzyl-7-fluor-5-(4-fluorphenyl)-1,3-dihydro-2,3,4-benzothiadiazepin-2,2-dioxid;
7-Fluor-5-(4-fluorphenyl)-3-(3-phenylpropyl)-1,3-dihydro-2,3,4-benzothiadiazepin-2,2-dioxid;
5-(3-Fluorphenyl)-7-chlor-3-methyl-1,3-dihydro-2,3,4-benzothiadiazepin-2,2-dioxid;
8-Cyano-5-(4-fluorphenyl)-3-methyl-1,3-dihydro-2,3,4-benzothiadiazepin-2,2-dioxid;
8-Fluor-5-(4-fluorphenyl)-3-methyl-1,3-dihydro-2,3,4-benzothiadiazepin-2,2-dioxid;
5-(4-Fluorphenyl)-8-chlor-3-methyl-1,3-dihydro-2,3,4-benzothiadiazepin-2,2-dioxid;
5-(4-Fluorphenyl)-3-methyl-8-trifluormethyl-1,3-dihydro-2,3,4-benzothiadiazepin-2,2-dioxid;
7,8-Dichlor-5-(4-fluorphenyl)-3-methyl-1,3-dihydro-2,3,4-benzothiadiazepin-2,2-dioxid;
9-(4-Fluorphenyl)-7-methyl-5H,7H-[1,2,5]thiadiazol[3,4-h]-2,3,4-benzothiadiazepin-6,6-dioxid;
7,8-Dichlor-3-ethyl-5-(4-fluorphenyl)-1,3-dihydro-2,3,4-benzothiadiazepin-2,2-dioxid;
7-Chlor-5-(4-fluorphenyl)-1,3-dimethyl-1,3-dihydro-2,3,4-benzothiadiazepin-2,2-dioxid;
7-Chlor-1-ethyl-5-(4-fluorphenyl)-3-methyl-1,3-dihydro-2,3,4-benzothiadiazepin-2,2-dioxid;
und/oder Enantiomere und/oder deren pharmazeutisch verträgliche Säureadditionssalze.

3. Verfahren zur Herstellung der Verbindungen mit der allgemeinen Formel (1) gemäß Anspruch 1, wobei R³ und R⁴ Wasserstoff sind und R¹, R², R⁵ und R⁶ in Anspruch 1 definiert sind, und deren pharmazeutisch geeigneten Säureadditionssalze durch Umsetzen der Verbindung mit der Formel (II) 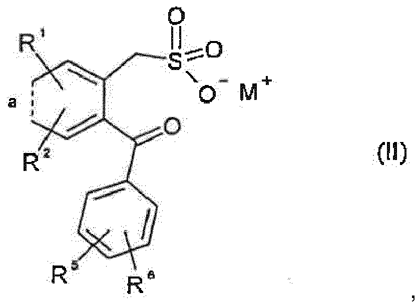 wobei R¹, R², R⁵ und R⁶ in Anspruch 1 definiert sind und M einwertige oder mehrwertige Metalle darstellt, mit Thionylchlorid und anschließendem Umsetzen der erhaltenen Verbindung mit der allgemeinen Formel (III) 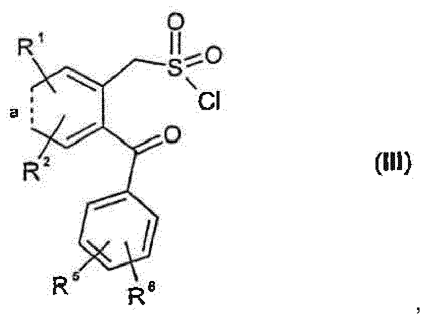 wobei R¹, R², R⁵ und R⁶ in Anspruch 1 definiert sind, mit Hydrazin.

4. Verfahren nach Anspruch 3 zur Herstellung der Verbindungen mit der allgemeinen Formel (I) gemäß Anspruch 1, wobei R⁴ Wasserstoff ist und R¹, R², R⁵, R⁶ und R³ in Anspruch 1 definiert sind, vorausgesetzt R³ ist nicht Wasserstoff, und deren pharmazeutisch geeigneten Säureadditionssalze durch Umsetzen der 2,3,4-Benzothiadiazepindioxidderivate mit der allgemeinen Formel (I), wobei R³ und R⁴ Wasserstoff sind, R¹, R², R⁵ und R⁶ in Anspruch 1 definiert sind, mit Stoffen, die zum Einführen einer Alkylgruppe, einer Alkenylgruppe oder einer Aralkylgruppe geeignet sind, in Gegenwart einer Base.

5. Verfahren nach Anspruch 3 zur Herstellung der Verbindungen mit der allgemeinen Formel (I) gemäß Anspruch 1, wobei R⁴ Wasserstoff ist und R¹, R², R⁵, R⁶ und R³ in Anspruch 1 definiert sind, vorausgesetzt R³ ist nicht Wasserstoff, und deren pharmazeutisch geeigneten Säureadditionssalze durch Umsetzen von Verbindungen mit der allgemeinen Formel (III) 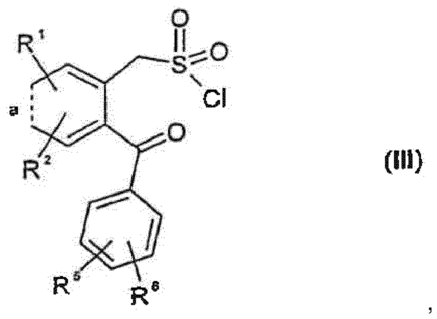 wobei R¹, R², R⁵ und R⁶ in Anspruch 1 definiert sind, mit einem substituierten Hydrazin mit der allgemeinen Formel (VI)
R³-NH-NH₂ (VI),
wobei R³ in Anspruch 1 definiert ist.

6. Verfahren nach Anspruch 3 zur Herstellung der Verbindungen mit der allgemeinen Formel (I) gemäß Anspruch 1, wobei R³ und R⁴ in Anspruch 1 definiert sind, vorausgesetzt R³ und R⁴ sind identisch und nicht Wasserstoff und R¹, R², R⁵ und R⁶ in Anspruch 1 definiert sind, und deren pharmazeutisch geeigneten Säureadditionssalze durch Umsetzen der 2,3,4-Benzothiadiazepindioxidderivate mit der allgemeinen Formel (I), wobei R³ und R⁴ Wasserstoff sind, R¹, R², R⁵ und R⁶ in Anspruch 1 definiert sind, mit Stoffen, die zum Einführen einer Alkylgruppe, einer Alkenylgruppe oder einer Aralkylgruppe geeignet sind, in Gegenwart einer Base.

7. Verfahren nach Anspruch 3 zur Herstellung der Verbindungen mit der allgemeinen Formel (I) gemäß Anspruch 1, wobei R³ und R⁴ in Anspruch 1 definiert sind, vorausgesetzt R³ und R⁴ sind nicht Wasserstoff und R¹, R², R⁵ und R⁶ in Anspruch 1 definiert sind, und deren pharmazeutisch geeigneten Säureadditionssalze durch Umsetzen der 2,3,4-Benzothiadiazepindioxidderivate mit der allgemeinen Formel (I), wobei R⁴ Wasserstoff ist, R¹, R², R⁵, R⁶ und R³ in Anspruch 1 definiert sind, vorausgesetzt R³ ist nicht Wasserstoff, mit Stoffen, die zum Einführen einer Alkylgruppe, einer Alkenylgruppe oder einer Aralkylgruppe geeignet sind, in Gegenwart einer Base.

8. Verbindungen mit der allgemeinen Formel (III) 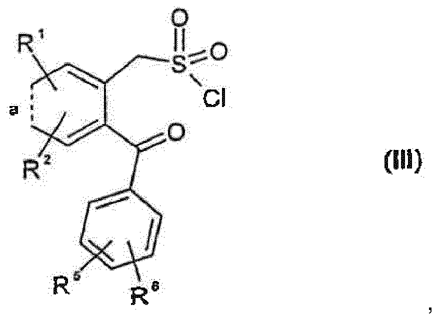 wobei R¹, R², R⁵ und R⁶ in Anspruch 1 definiert sind.

9. Verfahren zur Herstellung der Verbindungen mit der allgemeinen Formel (III) 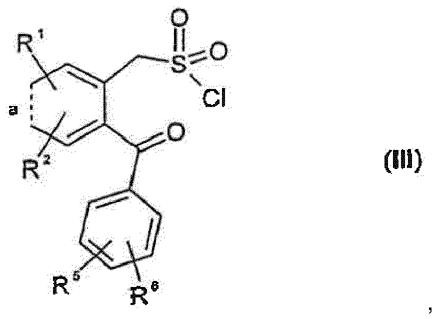 wobei R¹, R², R⁵ und R⁶ in Anspruch 1 definiert sind, das das Umsetzen der Verbindung mit der allgemeinen Formel (II) 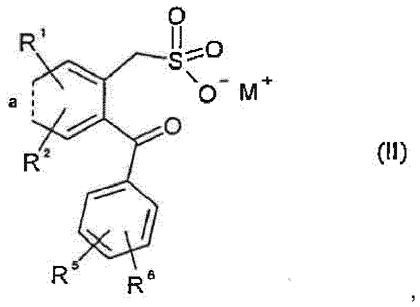 wobei R¹, R², R⁵ und R⁶ in Anspruch 1 definiert sind, mit Thionylchlorid umfasst.

10. Verbindungen mit der allgemeinen Formel (II) 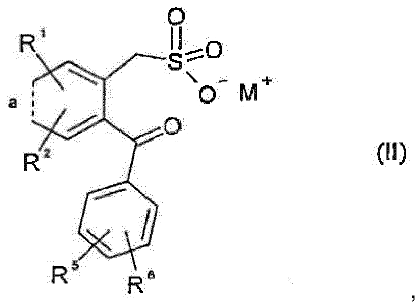 wobei R¹, R², R⁵ und R⁶ in Anspruch 1 definiert sind, M ein Metallatom, vorzugsweise ein Alkalimetallatom und besonders bevorzugt ein Natriumatom darstellt.

11. Verbindungen gemäß der Definition in Anspruch 10, Natrium[4-chlor-2-(4-fluorbenzoyl)-phenyl]-methansulfonat; Natrium[6-[(3,4-dimethoxyphenyl)-carbonyl]-2,1,3-benzothiadiazol-5-yl]methansulfonat; Natrium[6-[(4-fluorphenyl)-carbonyl]-2,1,3-benzothiadiazol-5-yl]-methansulfonat; Natrium-[6-[(4-methoxyphenyl)-carbonyl]-2,1,3-benzothiadiazol-5-yl]methansulfonat.

12. Verfahren zur Herstellung der Derivate mit der allgemeinen Formel (II) 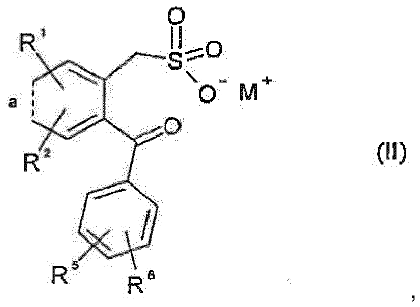 wobei R¹, R², R⁵ und R⁶ in Anspruch 1 definiert sind, das das Umsetzen der Verbindung mit der allgemeinen Formel (IV) 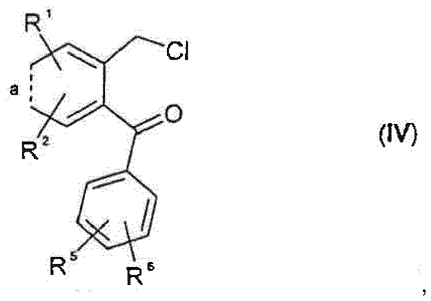 wobei R¹, R², R⁵ und R⁶ in Anspruch 1 definiert sind, mit Ammoniumsulfit oder Alkalisulfit in Gegenwart von Wasser oder eines mit Wasser nicht mischbaren Lösungsmittels, vorzugsweise in Aceton, Dioxane oder in einem 1 bis 4 Kohlenstoffatome umfassenden aliphatischen Alkohol oder in einer Mischung davon umfasst.

13. Verbindungen mit der allgemeinen Formel (I) gemäß der Definition in Anspruch 1 zur Verwendung als Arzneimittel.

14. Pharmazeutische Zusammensetzungen, die als wirksamen Bestandteil eine oder mehrere Verbindungen mit der allgemeinen Formel (I) gemäß der Definition in Anspruch 1 und/oder Enantiomere und/oder deren pharmazeutisch geeignete Säureadditionssalze in einer therapeutisch wirksamen Dosis zusammen mit einem oder mehreren pharmazeutisch verwendbaren Verdünnungsmitteln, Arzneistoffträgern und/oder inerten Trägerstoffen umfassen.

15. Verwendung der pharmazeutischen Zusammensetzung gemäß der Definition in Anspruch 14 zur Herstellung von Arzneimitteln zur Behandlung und/oder Vorbeugung von Krankheiten des zentralen Nervensystems.

16. Verbindungen mit der allgemeinen Formel (I) gemäß der Definition in Anspruch 1 zur Verwendung als Arzneimittel in der Behandlung und/oder Vorbeugung von Krankheiten des zentralen Nervensystems.

17. Verwendung von einer oder mehreren Verbindungen mit der allgemeinen Formel (I) gemäß der Definition in Anspruch 1 und/oder Enantiomeren und/oder deren pharmazeutisch geeigneten Säureadditionssalzen zur Herstellung eines Arzneimittels zur Behandlung von Erkrankungen des zentralen Nervensystems.

## Revendications

1. Composés de la Formule générale 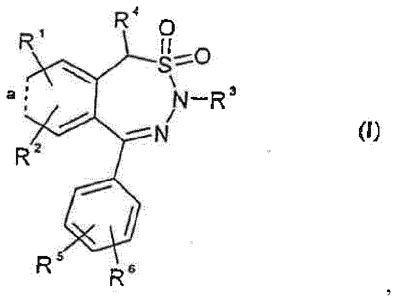 dans laquelle
'a' représente une liaison simple ou double,
R¹ et R² représentent indépendamment hydrogène, halogène, un groupe cyano, un groupe alkyle en C₁₋₆, un groupe alcoxy en C₁₋₆, un groupe trifluorométhyle ou un autre groupe alkyle en C₁₋₆ substitué par un ou plusieurs atomes d'halogène,
ou si
'a' représente une liaison simple,
R¹ et R² forment ensemble un groupe de la formule =N-S-N= relié aux atomes de la liaison 'a' en fonction de la Formule partielle (A), 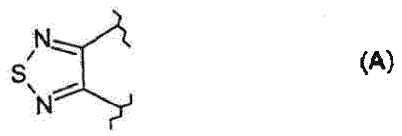
R³ et R⁴ représentent indépendamment hydrogène, un groupe alkyle en C₁₋₆, un groupe alkényle en C₁₋₆, un groupe cycloalkyle en C₃₋₆, un groupe aryle en C₁₋₆ ou un groupe aralkyle en C₁₋₆,
R⁵ et R⁶ représentent indépendamment hydrogène, halogène, un groupe cyano, un groupe alkyle en C₁₋₆, un groupe alcoxy en C₁₋₆, un groupe trifluorométhyle ou un autre groupe alkyle en C₁₋₆ substitué par un ou plusieurs atomes d'halogène,
énantiomères et sels d'addition acides pharmaceutiquement appropriés de ceux-ci.

2. Composé selon la revendication 1, sélectionné parmi
8-cyano-5-(4-fluoro-phényl)-1,3-dihydro-2,3,4-benzothiadiazépine-2,2-dioxyde ;
7-chloro-5-(3-fluoro-phényl)-1,3-dihydro-2,3,4-benzothiadiazépine-2,2-dioxyde ;
9-(4-méthyl-phényl)-5H,7H-[1,2,5]thiadiazolo[3,4-h]-2,3,4-benzothiadiazépine-6,6-dioxyde ;
9-(4-méthoxy-phényl)-5H,7H-[1,2,5]thiadiazolo[3,4-h]-[2,3,4]-benzothiadiazépine-6,6-dioxyde ;
9-(3,4-diméthoxy-phényl)-5H,7H-[1,2,5]thiadiazolo[3,4-h]-[2,3,4]-benzothiadiazépine-6,6-dioxyde ;
9-(4-fluoro-phényl)-5H,7H-[1,2,5]thiadiazolo[3,4-h]-[2,3,4]-benzothiadiazépine-6,6-dioxyde ;
7-chloro-5-(4-fluoro-phényl)-1,3-dihydro-2,3,4-benzothiadiazépine-2,2-dioxyde ;
7-fluoro-5-(4-fluoro-phényl)-1,3-dihydro-2,3,4-benzothiadiazépine-2,2-dioxyde ;
7,8-dichloro-5-(4-fluoro-phényl)-1,3-dihydro-2,3,4-benzothiadiazépine-2,2-dioxyde ;
7-chloro-5-(4-fluoro-phényl)-3-méthyl-1,3-dihydro-2,3,4-benzothiadiazépine-2,2-dioxyde ;
7-chloro-3-éthyl-5-(4-fluoro-phényl)-1,3-dihydro-2,3,4-benzothiadiazépine-2,2-dioxyde ;
7-chloro-5-(4-fluoro-phényl)-3-propyl-1,3-dihydro-2,3,4-benzothiadiazépine-2,2-dioxyde ;
7-chloro-5-(4-fluoro-phényl)-3-(prop-2-inyl)-1,3-dihydro-2,3,4-benzothiadiazépine-2,2-dioxyde ;
7-chloro-5-(4-fluoro-phényl)-3-isopropyl-1,3-dihydro-2,3,4-benzothiadiazépine-2,2-dioxyde ;
7-chloro-5-(4-fluoro-phényl)-3-(3-méthyl-but-2-ényl)-1,3-dihydro-2,3,4-benzothiadiazépine-2,2-dioxyde ;
7-chloro-5-(4-fluoro-phényl)-3-(3-méthyl-butyl)-1,3-dihydro-2,3,4-benzothiadiazépine-2,2-dioxyde ;
3-benzyl-7-chloro-5-(4-fluoro-phényl)-1,3-dihydro-2,3,4-benzothiadiazépine-2,2-dioxyde ;
7-fluoro-5-(4-fluoro-phényl)-3-méthyl-1,3-dihydro-2,3,4-benzothiadiazépine-2,2-dioxyde ;
7-fluoro-3-éthyl-5-(4-fluoro-phényl)-1,3-dihydro-2,3,4-benzothiadiazépine-2,2-dioxyde ;
3-butyl-7-fluoro-5-(4-fluoro-phényl)-1,3-dihydro-2,3,4-benzothiadiazépine-2,2-dioxyde ;
7-fluoro-5-(4-fluoro-phényl)-3-(3-méthyl-but-2-ényl)-1,3-dihydro-2,3,4-benzothiadiazépine-2,2-dioxyde ;
7-fluoro-5-(4-fluoro-phényl)-3-(3-méthyl-butyl)-1,3-dihydro-2,3,4-benzothiadiazépine-2,2-dioxyde ;
7-fluoro-5-(4-fluoro-phényl)-3-hexyl-1,3-dihydro-2,3,4-benzothiadiazépine-2,2-dioxyde ;
3-benzyl-7-fluoro-5-(4-fluoro-phényl)-1,3-dihydro-2,3,4-benzothiadiazépine-2,2-dioxyde ;
7-fluoro-5-(4-fluoro-phényl)-3-(3-phényl-propyl)-1,3-dihydro-2,3,4-benzothiadiazépine-2,2-dioxyde ;
5-(3-fluoro-phényl)-7-chloro-3-méthyl-1,3-dihydro-2,3,4-benzothiadiazépine-2,2-dioxyde ;
8-cyano-5-(4-fluoro-phényl)-3-méthyl-1,3-dihydro-2,3,4-benzothiadiazépine-2,2-dioxyde ;
8-fluoro-5-(4-fluoro-phényl)-3-méthyl-1,3-dihydro-2,3,4-benzothiadiazépine-2,2-dioxyde ;
5-(4-fluoro-phényl)-8-chloro-3-méthyl-1,3-dihydro-2,3,4-benzothiadiazépine-2,2-dioxyde ;
5-(4-fluoro-phényl)-3-méthyl-8-trifluorométhyl-1,3-dihydro-2,3,4-benzothiadiazépine-2,2-dioxyde ;
7,8-dichloro-5-(4-fluoro-phényl)-3-méthyl-1,3-dihydro-2,3,4-benzothiadiazépine-2,2-dioxyde ;
9-(4-fluoro-phényl)-7-méthyl-5H,7H-[1,2,5]-thiadiazolo[3,4-h][2,3,4]-benzothiadiazépine-6,6-dioxyde ;
7,8-dichloro-3-éthyl-5-(4-fluoro-phényl)-1,3-dihydro-2,3,4-benzothiadiazépine-2,2-dioxyde ;
7-chloro-5-(4-fluoro-phényl)-1,3-diméthyl-1,3-dihydro-2,3,4-benzothiadiazépine-2,2-dioxyde ;
7-chloro-1-éthyl-5-(4-fluoro-phényl)-3-méthyl-1,3-dihydro-2,3,4-benzothiadiazépine-2,2-dioxyde
et/ou énantiomères et/ou sels d'addition acides pharmaceutiquement acceptables de celui-ci.

3. Procédé pour la préparation des composés de la Formule générale (I) selon la revendication 1,
dans lequel R³ et R⁴ sont hydrogène, et R¹, R², R⁵ et R⁶ sont définis à la revendication 1, et de sels d'addition acides pharmaceutiquement appropriés de ceux-ci, en faisant réagir le composé de la Formule (II) 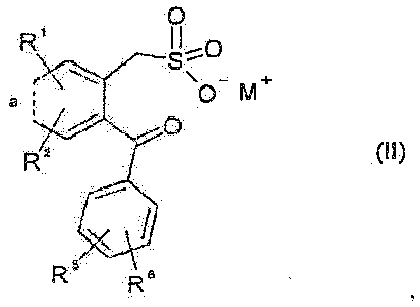 dans laquelle R¹, R², R⁵ et R⁶ sont définis à la revendication 1 et M représente des métaux monovalents ou multivalents, avec du chlorure de thionyle et par réaction subséquente du composé obtenu de la Formule générale (III) 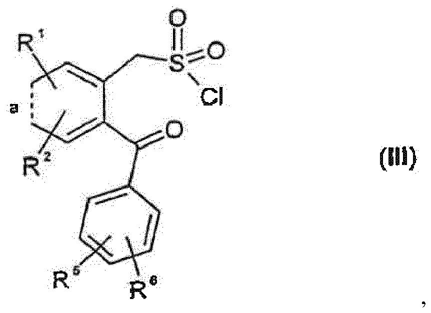 dans laquelle R¹, R², R⁵ et R⁶ sont définis à la revendication 1, avec de l'hydrazine.

4. Procédé selon la revendication 3 pour la préparation des composés de la Formule générale (I) selon la revendication 1,
dans lequel R⁴ est hydrogène et R¹, R², R⁵ et R⁶ sont définis à la revendication 1 à condition que R³ soit différent d'hydrogène, et de sels d'addition acides pharmaceutiquement appropriés de ceux-ci, en faisant réagir les dérivés de 2,3,4-benzothiadiazépine-dioxyde de la Formule générale (I) dans laquelle R³ et R⁴ sont hydrogène, R¹, R², R⁵ et R⁶ sont définis à la revendication 1, avec des agents convenant à introduire un groupe alkyle, groupe alkényle ou groupe aralkyle en présence d'une base.

5. Procédé selon la revendication 3 pour la préparation des composés de la Formule générale (I) selon la revendication 1,
dans lequel R⁴ est hydrogène et R¹, R², R⁵ et R⁶ sont définis à la revendication 1 à condition que R³ soit différent d'hydrogène, et de sels d'addition acides pharmaceutiquement appropriés de ceux-ci, en faisant réagir des composés de la formule générale (III) 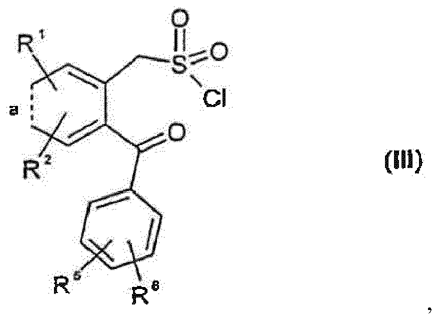 dans laquelle R¹, R², R⁵ et R⁶ sont définis à la revendication 1, avec une hydrazine substituée de formule générale (VI)
R³-NH-NH₂ (VI),
dans laquelle R³ est défini à la revendication 1.

6. Procédé selon la revendication 3 pour la préparation des composés de la Formule générale (I) selon la revendication 1,
dans lequel R³ et R⁴ sont définis à la revendication 1 à condition que R³ et R⁴ soient identiques et différents d'hydrogène, et R¹, R², R⁵ et R⁶ sont définis à la revendication 1, et de sels d'addition acides pharmaceutiquement appropriés de ceux-ci, en faisant réagir les dérivés de 2,3,4-benzothiadiazépine-dioxyde de la Formule générale (I) dans laquelle R³ et R⁴ sont hydrogène, R¹, R², R⁵ et R⁶ sont définis à la revendication 1, avec des agents convenant à introduire un groupe alkyle, groupe alkényle ou groupe aralkyle en présence d'une base.

7. Procédé selon la revendication 3 pour la préparation des composés de la Formule générale (I) selon la revendication 1,
dans lequel R³ et R⁴ sont définis à la revendication 1 à condition que R³ et R⁴ soient différents d'hydrogène, et R¹, R², R⁵ et R⁶ sont définis à la revendication 1, et de sels d'addition acides pharmaceutiquement appropriés de ceux-ci, en faisant réagir les dérivés de 2,3,4-benzothiadiazépine-dioxyde de la Formule générale (I) dans laquelle R⁴ est hydrogène, R¹, R², R⁵ et R⁶ sont définis à la revendication 1 à condition que R³ soit différent d'hydrogène, avec des agents convenant à introduire un groupe alkyle, groupe alkényle ou groupe aralkyle en présence d'une base.

8. Composés de Formule générale (III) 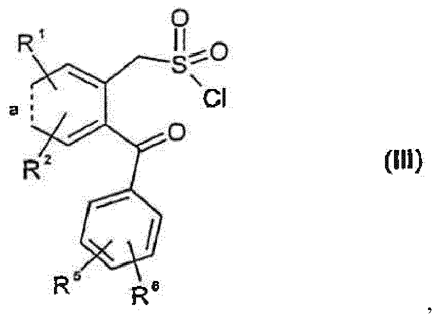 dans laquelle R¹, R², R⁵ et R⁶ sont définis à la revendication 1.

9. Procédé pour la préparation des composés de Formule générale (III) 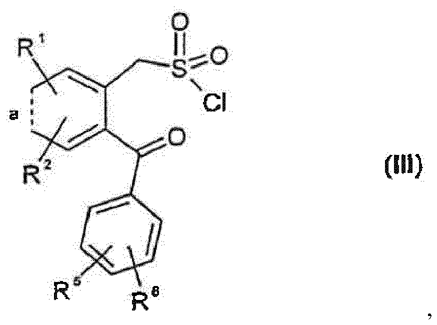 dans laquelle R¹, R², R⁵ et R⁶ sont définis à la revendication 1, qui comprend le fait de faire réagir le composé de Formule générale (II) 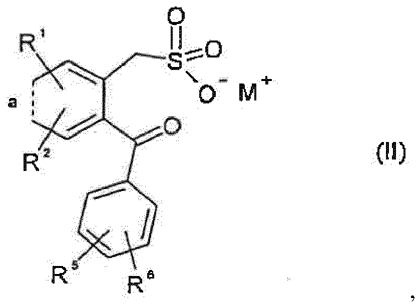 dans laquelle R¹, R², R⁵ et R⁶ sont définis à la revendication 1, avec du chlorure de thionyle.

10. Composés de Formule générale (II) 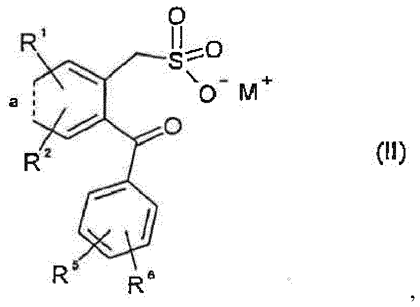 dans laquelle R¹, R², R⁵ et R⁶ sont définis à la revendication 1, M représente un atome métallique, de préférence un atome de métal alcalin, plus préférablement un atome de sodium.

11. Composés selon la revendication 10
[4-chloro-2-(4-fluoro-benzoyl)-phényl]-méthanesulfonate sodique ;
[6-[(3,4-diméthoxy-phényl)-carbonyl]-2,1,3-benzothiadiazole-5-yl]-méthanesulfonate sodique ;
[6-[(4-fluoro-phényl)-carbonyl]-2,1,3-benzothiadiazole-5-yl]-méthanesulfonate sodique ;
[6-[(4-méthoxy-phényl)-carbonyl]-2,1,3-benzothiadiazole-5-yl]-méthanesulfonate sodique.

12. Procédé pour la préparation des dérivés de Formule générale (II) 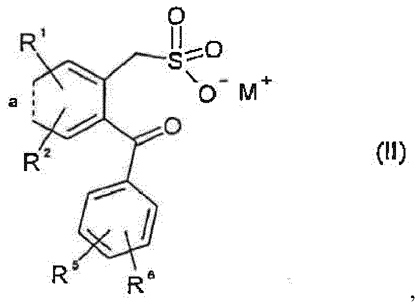 dans laquelle
R¹, R², R⁵ et R⁶ sont définis à la revendication 1, qui comprend le fait de faire réagir le composé de Formule générale (IV) 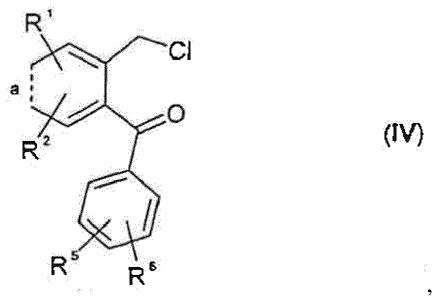 dans laquelle R¹, R², R⁵ et R⁶ sont définis à la revendication 1, avec du sulfite d'ammonium ou sulfite alcalin en présence d'eau ou d'un solvant immiscible dans l'eau, de préférence dans de l'acétone, du dioxane ou dans un alcool aliphatique comprenant 1 à 4 atomes de carbone ou dans le mélange de ceux-ci.

13. Composés de la Formule générale (I) selon la revendication 1 destinés à être utilisés en tant que médicament.

14. Compositions pharmaceutiques comprenant en tant qu'ingrédient actif un ou plusieurs composés de Formule générale (I) selon la revendication 1 et/ou énantiomères et/ou sels d'addition acides pharmaceutiquement appropriés de ceux-ci en une dose efficace du point de vue thérapeutique avec un ou plusieurs diluant, excipient et/ou support inerte pharmaceutiquement applicables.

15. Utilisation d'une composition pharmaceutique selon la revendication 14 pour la préparation de médicaments pour le traitement et/ou la prévention de maladies du système nerveux central.

16. Composés de la Formule générale (I) selon la revendication 1 destinés à être utilisés en tant que médicament dans le traitement et/ou la prévention de maladies du système nerveux central.

17. Utilisation d'un ou plusieurs composés de Formule générale (I) selon la revendication 1 et/ou énantiomères et/ou sels d'addition acides pharmaceutiquement appropriés de ceux-ci pour la préparation d'un médicament pour le traitement de troubles du système nerveux central.
